# EUROPEAN PATENT APPLICATION

(11) **EP 4 144 296 A1**
(43) Date of publication of application: **08.03.2023**
(21) Application number: 21814520.9
(22) Date of filing: 25.05.2021
(51) Int. Cl.: A61B 6/00, A61B 6/03

(54) **STATE DETECTION METHOD AND SYSTEM FOR IMAGING DEVICE**

(30) Priority: 25.05.2020 CN 202010448695; 25.05.2020 CN 202010448693; 29.06.2020 CN 202010604518; 29.06.2020 CN 202010604531; 02.07.2020 CN 202010626175
(71) Applicant: Shanghai United Imaging Healthcare Co., Ltd., Shanghai 201807 (CN)
(72) Inventor: LYU, Xinyu, Shanghai 201807 (CN); XUE, Manquan, Shanghai 201807 (CN); CHU, Shaoping, Shanghai 201807 (CN); WANG, Xuming, Shanghai 201807 (CN)
(74) Representative: Wang, Bo
(86) International application number: PCT/CN2021/095840
(87) International publication number: WO 2021/238929

(57) **Abstract**

The embodiments of the present disclosure provide a state detection method and system for an imaging device. The method includes: obtaining a first background event of a crystal of a detector of the imaging device, the first background event being related to an inherent radiating particle of the crystal; correcting a crystal position look-up table based on the first background event; correcting an energy state of the imaging device; obtaining the second background event of the crystal, the second background event being related to the inherent radiating particle of the crystal; and correcting a state of time of flight of the detector based on the first background event and the second background event.

## Description

### CROSS-REFERENCE TO RELATED APPLICATIONS

The present application claims priority to Chinese Application No. 202010448695.5, filed on May 25, 2020, Chinese Application No. 202010448693.6, filed on May 25, 2020, Chinese Application No. 202010604518.1, filed on June 29, 2020, Chinese Application No. 202010604531.7, filed on June 29, 2020, and Chinese Application No. 202010626175.9, filed on July 2, 2020, the entire contents of which are incorporated herein by reference.

### TECHNICAL FIELD

The present disclosure relates to the field of imaging technologies, and in particular, to a state detection method and system of an imaging device.

### BACKGROUND

Imaging technology has been widely used in biological detection, medical diagnosis, and other fields. Taking Positron Emission Computed Tomography (PET) as an example, Positron Emission Computed Tomography (PET) is a relatively advanced clinical examination imaging technology in the field of nuclear medicine. In PET imaging, radionuclides are injected into the target object, so that certain substances in the target object (generally necessary substances in the metabolism of biological life, such as glucose, protein, nucleic acid, fatty acid, etc.) are marked with short-lived radionuclides. Elements (such as F18, carbon 11, etc.). After being injected into the human body, the radionuclide releases positrons during the decay process. A positron annihilates after encountering an electron after traveling a few tenths of a millimeter to a few millimeters, resulting in a pair of photons with an energy of 511 keV in opposite directions. The photon signal is captured by a highly sensitive camera and corrected for scattering and random information by a computer. Through the same analysis and processing of different positrons, a three-dimensional image reflecting the accumulation of radionuclides in the target object may be obtained, so as to achieve the purpose of diagnosis. PET scanning has the characteristics of high sensitivity, high specificity, and good safety, so it is widely used in the medical field. In order to improve the accuracy of the data collected by the PET device and the quality of the PET image, it is necessary to detect and/or correct the state of the PET device.

Therefore, it is desirable to provide a state detection method and system for an imaging device, such as a PET imaging device.

### SUMMARY

An aspect of the present disclosure provides a state detection method of an image-forming device. The method includes: obtaining a first background event of a crystal of a detector of an imaging device, the first background event is related to an inherent radiating particle of the crystal; correcting a crystal position look-up table based on the first background event; correcting an energy state of the imaging device; obtaining a second background event of the crystal, the second background event is related to the inherent radiating particle of the crystal; and correcting a state of time of flight of the detector based on the first background event and the second background event.

In some embodiments, the obtaining the first background event of the crystal of the detector of the imaging device includes: obtaining, based on a preset energy window, the first background event of the inherent radiating particle of the crystal received by the detector; and the correcting a crystal position look-up table based on the first background event includes: determining a single-event image based on the first background event; and correcting the crystal position look-up table based on the single-event image.

In some embodiments, the correcting an energy state of the imaging device comprises: correcting the energy state of the imaging device based on the first background event.

In some embodiments, the correcting the energy state of the imaging device includes obtaining a third background event of the crystal, wherein the third background event is related to the inherent radiating particle of the crystal, and the third background event includes a background single event or a background coincidence event of the inherent radiating particle of the crystal received by the detector; and correcting the energy state of the imaging device based on the third background event.

In some embodiments, the correcting an energy state of the imaging device comprises: generating an energy spectrogram based on energy information of the first background event or the third background event; determining at least one peak position in the energy spectrogram; determining an energy correction state of the imaging device based on the at least one peak position in the energy spectrogram and a corrected peak position corresponding to the at least one peak position; and correcting the energy state of the imaging device based on the energy correction state.

In some embodiments, the correcting the energy state of the imaging device comprises: determining, based on the first background event or the third background event, at least two energy peak values associated with a nuclide decay of the crystal and ADC values corresponding to the at least two energy peak values; and determining an energy scale curve of the imaging device based on the at least two energy peak values and the ADC values corresponding to the at least two energy peak values.

In some embodiments, the correcting a state of time of flight of the detector based on the first background event and the second background event includes: determining a measured time of flight based on the first background event and the second background event; and correcting a time of flight of the detector based on the state of time of flight of the imaging device reflected by the measured time of flight.

In some embodiments, the method further includes: generating an event time chart based on at least one of the first background event or the second background event; determine a corresponding relationship between TDC values and time based on the event time chart; and determining a TDC scale curve of the imaging device based on the corresponding relationship.

In some embodiments, the method further includes: obtaining a fourth background event of the crystal, the fourth background event being related to the inherent radiating particle of the crystal; generating an event time chart based on the fourth background event; determining a corresponding relationship between a TDC value and a time based on the event time chart; and determining a TDC scale curve of the imaging device based on the corresponding relationship.

In some embodiments, the method further comprises: determining, based on the first background event and the second background event, the measured time of flight and a theoretical time of flight; and performing a time-synchronization on a detector module of the detector based on the measured time of flight and the theoretical time of flight.

An aspect of the present disclosure provides a crystal position look-up table correction method. The method includes: obtaining a background event of a crystal of a detector of an imaging device, the background event being related to an inherent radiating particle of the crystal; determining a single-event image based on the background event; and correcting the crystal position look-up table of the imaging device based on the single-event image.

In some embodiments, the obtaining a background event of a crystal of a detector of an imaging device comprises: determining an energy window of the detector of the imaging device, a value range of the energy window being within a clinical threshold value range of the energy window of the imaging device; and obtaining, based on the energy window, the background event of the inherent radiating particle of the crystal received by the detector.

In some embodiments, the value range of the energy window is within a clinical threshold value range of the energy window of the imaging device.

In some embodiments, the determining a single-event image based on the background event includes: determining a single-characteristic-energy-peak event based on the background event; generating the single-event image based on the single-characteristic-energy-peak event.

In some embodiments, the single-characteristic-energy-peak event comprises an event of 597 keV at least one photon received by the detector.

In some embodiments, the calibrating the crystal position look-up table of the imaging device based on the single-event image includes: obtaining, based on the single-event image, the position index of the crystal in the crystal position look-up table Corresponding pixel distribution in the single-event image; correcting the crystal position look-up table based on the corresponding pixel distribution of the crystal position index in the single-event image.

In some embodiments, the method further includes determining whether a deviation related to the crystal position look-up table of the imaging device has occurred based on the background event.

An aspect of the present disclosure provides an energy correction state detection method. The method includes: obtaining a background event of a crystal of a detector of an imaging device, the background event being related to an inherent radiating particle of the crystal; generating an energy spectrogram based on energy information of the background event; determining at least one peak position in the energy spectrogram; and determining the energy correction state based on the at least one peak position in the energy spectrogram and at least one corrected peak position corresponding to the at least one peak position in the energy spectrogram.

In some embodiments, the obtaining a background event of a crystal of a detector of an imaging device includes: determining an event-obtaining mode of the detector; obtaining the background events of the inherent radiating particle of the crystal of the device received by the detector based on the event-obtaining mode.

In some embodiments, the event-obtaining mode includes a single event mode, and the generating an energy spectrogram based on the energy information of the background event includes: generating the energy spectrogram based on the background received by the detector in the single event mode. The energy spectrogram includes at least one of a peak value of a full energy peak or a peak value of a single energy peak.

In some embodiments, the event-obtaining mode includes a coincidence event mode, and obtaining the background events of the inherent radiating particle of the crystal of the detector received by the detector includes: obtaining the background events of the inherent radiating particle of the crystal of the detector received by the detector in the coincident event mode based on the preset time window and/or the preset energy window.

In some embodiments, the generating an energy spectrogram based on the energy information of the background event includes: filtering the particle energy information based on the particle arrival time of the background event; generating the energy spectrum based on the filtered particle energy information. The energy spectrogram includes a peak value of a single energy peak.

In some embodiments, the range of the preset time window is no less than the range of the clinical time window threshold value of the imaging device.

In some embodiments, the determining the energy correction state based on the at least one peak position in the energy spectrogram and the at least one corrected peak position corresponding to the at least one peak position in the energy spectrogram includes: determining a ratio of a position of the peak value of the energy spectrogram to the position of the at least one corrected peak position; determining, based on the ratio, whether the energy correction state of the imaging device is abnormal.

In some embodiments, the at least one peak position in the energy spectrogram includes at least one of a peak position of a full energy peak or a peak position of a single energy peak.

In some embodiments, the at least one corrected peak position corresponds to a peak position of an energy spectrogram of 511 keV at least one photon.

An aspect of the present disclosure provides a method for determining an energy scale curve. The method includes: obtaining a background event of a crystal of a detector of an imaging device, the background event being related to an inherent radiating particle of the crystal; determining, based on the background event, at least two energy peak values associated with a nuclide decay of the crystal and ADC values corresponding to the at least two energy peak values; and determining the energy scale curve based on the at least two energy peak values and the ADC values corresponding to the at least two energy peak values.

In some embodiments, the determining at least two energy peak values associated with a nuclide decay of the crystal and ADC values corresponding to the at least two energy peak values based on the background event includes: determining decay characteristic energy peak values of the nuclide of the crystal based on the background event; obtaining an ADC value corresponding to the decay characteristic energy peak values by performing an analog-to-digital conversion on the decay characteristic energy peak values of the nuclide; and selecting the at least two energy peak values in the decay characteristic energy peak values and the ADC values corresponding to the at least two energy peak values.

In some embodiments, the at least two energy peak values include 307 keV and 597 keV energy peak values; and the ADC values corresponding to the at least two energy peak values include ADC values corresponding to the 307 keV and 597 keV energy peak values.

In some embodiments, the determining the energy scale curve based on the at least two energy peak values and the ADC values corresponding to the at least two energy peak values includes: by comparing the at least two energy peak values and the ADC values with the at least two energy peak values. The ADC values corresponding to the at least two energy peak values are interpolated and fitted to determine the energy scale curve.

In some embodiments, the method further includes: determining, based on the energy scale curve, an ADC value corresponding to any energy peak on the energy scale curve.

In some embodiments, the method further includes: determining an ADC value corresponding to the 511 keV energy peak value based on the energy scale curve.

In some embodiments, the method further includes: correcting the energy of the at least one particle received by the detector based on the ADC value corresponding to the 511 keV energy peak.

An aspect of the present disclosure provides a time-synchronization method. The method includes: obtaining at least one background coincidence event of a crystal of a detector of an imaging device, the at least one background coincidence event being related to an inherent radiating particle of the crystal; determining a measured time of flight and a theoretical time of flight based on information of the at least one background coincidence event; and performing a time-synchronization on the detector based on the measured time of flight and the theoretical time of flight.

In some embodiments, the information of the at least one background coincidence event includes: the information of the at least one background coincidence event includes: arrival time of two particles reaching the detector of each background coincidence event of the at least one background coincidence event and crystal positions corresponding to the two particles; the determining a measured time of flight and a theoretical time of flight based on information of the at least one background coincidence event includes: determining a measured time of flight of each background coincidence event based on the arrival time of the two particles of the background coincidence event; and determining a theoretical time of flight of each background coincidence event based on the crystal positions corresponding to the two particles of the background coincidence event.

In some embodiments, performing time synchronization on the detector based on the measured time of flight and the theoretical time of flight includes: determining a time difference based on the measured time of flight and the theoretical time of flight; based on the time difference, time synchronization is performed on the detectors.

In some embodiments, the determining the time difference based on the measured time of flight and the theoretical time of flight includes: based on a plurality of measured values of time of flight and a plurality of theoretical values of time of flight received by any pair of detector modules of the detector time, determining the difference between the plurality of measured values of time of flight and the plurality of theoretical values of time of flight; determining the average value of the difference between the plurality of measured values of time of flight and the plurality of theoretical values of time of flight as the time difference.

In some embodiments, performing time synchronization on the detector based on the time difference includes: in response to the time difference being greater than or equal to one clock cycle, performing time synchronization on detector modules of the detector.

In some embodiments, the time synchronization of the detector includes: adjusting a value of a counter of one detector module in any pair of detector modules of the detector, and the adjusted value of the counter is determined as the time reference standard.

In some embodiments, the method further includes: determining any one detector module in the pair of detector modules corresponding to the adjusted counter as a reference module, and performing the time-synchronization on other detector modules in the detector except the pair of detector modules based on the reference module.

An aspect of the present disclosure provides a state of time of flight detection method. The method includes: obtaining a background coincidence event of a crystal of a detector of an imaging device, the background coincidence event being related to an inherent radiating particle of the crystal; determining a measured time of flight and a theoretical time of flight based on information of the background coincidence event; and determining a state of time of flight of the imaging device based on the measured time of flight and the theoretical time of flight, wherein the state of time of flight reflects whether the crystal drifts.

In some embodiments, the obtaining at least one background coincidence event of a crystal of a detector of the imaging device includes: determining a time window and an energy window of the imaging device; obtaining the detector reception based on the time window and the energy window. The resulting background of inherent radiating particle of the crystal is consistent with the event and its associated information.

In some embodiments, the range of the time window is no less than the range of the clinical time window threshold value of the imaging device; the value range of the energy window is no less than the clinical threshold value range of the energy window of the imaging device.

In some embodiments, the information of the at least one background coincidence event includes: the arrival time of two particles in each of the at least one background coincidence event reaching the detector and the corresponding crystal positions; the Based on the information of the at least one background coincidence event, determining the measurement time of flight and the theoretical time of flight includes: based on the arrival times of the two particles of each background coincidence event, determining the measurement of each background coincidence event time of flight; and determining the theoretical time of flight of each background coincidence event based on the crystal positions corresponding to the two particles of each background coincidence event.

In some embodiments, the determining the time-of-flight state of the imaging device based on the measured time of flight and the theoretical time of flight comprises: in response to a difference between the measured time of flight and the theoretical time of flight exceeding a threshold, A crystal drift is determined corresponding to the at least one background coincidence event.

In some embodiments, the method further includes: correcting the time of flight of the detector based on the information of the at least one background coincidence event to obtain the corrected time of flight.

In some embodiments, the correcting the time of flight of the detector based on the information of the at least one background coincidence event to obtain the corrected time of flight includes: obtaining an energy-time mapping relationship, wherein the energy-time mapping relationship reflects a corresponding relationship between a particle energy and a time-of-flight offset; determining, based on the energy-time mapping relationship, a time-of-flight offset corresponding to the energy of the two particles in each background coincidence event; and correcting the time of flight of the detector based on the time-of-flight offset to obtain the corrected time of flight.

Another aspect of the present disclosure provides a state detection device of an image forming device. The device includes: an obtaining module for obtaining a first background event and a second background event of a crystal of a detector of the imaging device, the first background event and the second background event being related to the inherent radiating particle of the crystal; a correction module for correcting the crystal position look-up table based on the first background event; correcting the energy state of the imaging device; and based on the first background event The background event and the second background event are used to correct the state of a time of flight of the detector.

Another aspect of the present disclosure provides a computer device including a memory, a processor, and a computer program stored on the memory and executable on the processor, wherein the processor executes the computer program implement the method described above.

Another aspect of the present disclosure provides a computer-readable storage medium on which a computer program is stored, characterized in that, when the program is executed by a processor, the aforementioned-method is implemented.

### BRIEF DESCRIPTION OF THE DRAWINGS

The present disclosure will be further described in the form of exemplary embodiments, which will be described in detail by the accompanying drawings. These embodiments are not restrictive. In these embodiments, the same number represents the same structure, wherein:
FIG. 1 is a schematic diagram of an application scenario of a state detection method of an exemplary imaging device according to some embodiments of the present disclosure;
FIG. 2 is a schematic diagram of exemplary hardware and/or software of a computing device according to some embodiments of the present disclosure;
FIG. 3 is an exemplary block diagram of a state correction device of an imaging device according to some embodiments of the present disclosure;
FIG. 4 is an exemplary flowchart of a state detection method of an imaging device according to some embodiments of the present disclosure;
FIG. 5 is an exemplary schematic diagram of a PET device receiving at least one particle according to some embodiments of the present disclosure;
FIG. 6 is an exemplary block diagram of a correction device of a crystal position look-up table according to some embodiments of the present disclosure;
FIG. 7 is an exemplary flowchart of a crystal position look-up table correction method according to some embodiments of the present disclosure;
FIG. 8A-8C are exemplary schematic diagrams of a correction method of a crystal position look-up table according to some embodiments of the present disclosure;
FIG. 9 is an exemplary block diagram of an energy state detection device according to some embodiments of the present disclosure;
FIG. 10 is an exemplary flowchart of an energy state detection method according to some embodiments of the present disclosure;
FIG. 11 is a schematic diagram of an energy spectrogram formed by a single-event mode according to some embodiments of the present disclosure;
FIG. 12 is a schematic diagram of an energy spectrogram formed by a coincidence event mode according to some embodiments of the present disclosure;
FIG. 13 is an exemplary block diagram of a device for determining an energy scale curve according to some embodiments of the present disclosure;
FIG. 14 is an exemplary flowchart of a process for determining an energy scale curve according to some embodiments of the present disclosure;
FIG. 15 is an exemplary block diagram of a time-synchronization device according to some embodiments of the present disclosure;
FIG. 16 is an exemplary flowchart of a time-synchronization method according to some embodiments of the present disclosure;
FIG. 17 is a schematic diagram of a detector module receiving at least one photon according to some embodiments of the present disclosure;
FIG. 18 is an exemplary block diagram of a time-of-flight state detection device according to some embodiments of the present disclosure;
FIG. 19 is an exemplary flowchart of a method for detecting a state of time of flight according to some embodiments of the present disclosure;
FIG. 20 is an exemplary schematic diagram of an event moment spectrum according to some embodiments of the present disclosure;
FIG. 21 is an exemplary schematic diagram of a corresponding relationship between TDC values and time according to some embodiments of the present disclosure.

### DETAILED DESCRIPTION

In order to more clearly explain the technical scheme of the embodiments of the present disclosure, the following will briefly introduce the drawings that need to be used in the description of the embodiments. Obviously, the drawings in the following description are only some examples or embodiments of the present disclosure. For those skilled in the art, the present disclosure can also be applied to other similar scenarios based on these drawings without creative work. Unless it is obvious from the language environment or otherwise stated, the same label in the figure represents the same structure or operation.

It should be understood that the "system," "device," "unit" and/or "module" used herein is a method for distinguishing different components, elements, parts, or assemblies at different levels. However, if other words can achieve the same purpose, they can be replaced by other expressions.

As shown in the description and the claims, unless the context expressly indicates exceptions, the words "a," "an," "the," "one," and/or "this" do not specifically refer to the singular, but may also include the plural; and the plural forms may be intended to include the singular forms as well, unless the context clearly indicates otherwise. Generally speaking, the terms "include" only indicate that the steps and elements that have been clearly identified are included, and these steps and elements do not constitute an exclusive list. Methods or equipment may also include other steps or elements.

A flowchart is used in this disclosure to explain the operation performed by the system based on the embodiment of the present disclosure. It should be understood that the previous or subsequent operations are not necessarily performed accurately in order. Instead, the steps may be processed in reverse order or simultaneously. At the same time, you can add other operations to these processes, or remove one or more steps from these processes.

A PET device may generate an image by detecting a pair of γ photons produced by positron annihilation. In some embodiments, an accuracy of data collected by the PET device, a quality of a PET image, etc., may be improved by periodically or real-time detecting and correcting a state of the PET device. In some embodiments, a radioactive rod source (barrel source) may be used to implement a state detection of the PET device. However, the state detection under the presence of the radioactive source may cause a certain degree of radiation damage to a user (for example, a medical staff) due to the existence of radioactive sources, and increase a radiation dose received by an operator. The operation method may be complicated, and may only be applied on regular detections.

The method for detecting a state of an imaging device provided by the embodiments of the present disclosure may detect the state of the imaging device based on an inherent radioactive phenomenon of a crystal of a detector of the imaging device and may detect the state of the imaging device without an additional radioactive rod source (barrel source). The method of detecting the state of the imaging device may simplify the detection process, reduce the radiation dose received by an operator, and save the cost of purchasing radioactive sources. The operation of the method is also simple. In addition, this method may be used for performing a state detection when the PET device is in an idle state, which means the state detection may be performed at any idle time and may be performed multiple times repeatedly. This may be convenient for increasing the frequency of detection and an abnormal state of the device may be discovered timely. so the method may be more flexible.

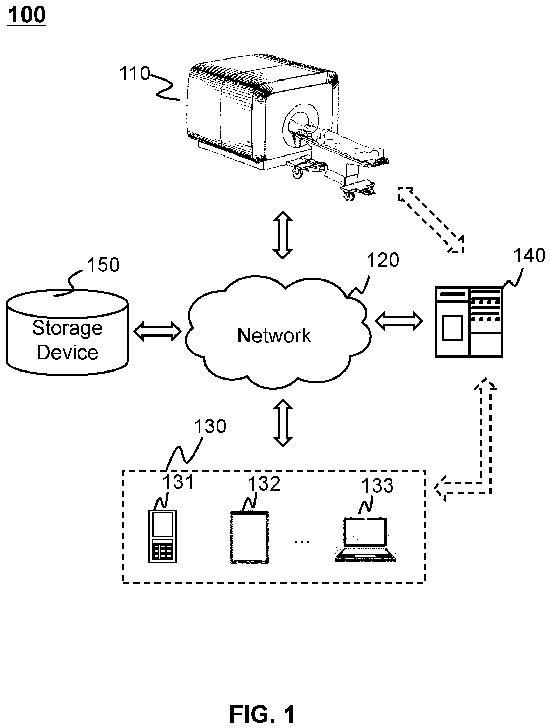

FIG. 1 is a schematic diagram of an application scenario of a state detection method of an exemplary imaging device according to some embodiments of the present disclosure.

As shown in FIG. 1, the system 100 for state detection of an imaging device may include an imaging device 110, a network 120, a terminal(s) 130, a processing device 140, and a storage device 150. Various components in the system 100 may be connected to each other through the network 120. For example, the imaging device 110 and the terminal(s) 130 may be connected or communicated through the network 120. As another example, the imaging device 110 and the storage device 150 may be connected or communicated through the network 120.

The imaging device 110 may be configured to scan a target object in the detection area to obtain scan data of the target object. In some embodiments, the target object may include a biological object and/or a non-biological object. For example, the target object may include a specific part of the body, such as the head, chest, abdomen, etc., or a combination thereof. As another example, the target object may be animate or inanimate organic and/or inorganic matter.

In some embodiments, the imaging device 110 may be a non-invasive biomedical imaging device configured for disease diagnosis or research purposes. For example, the imaging device 110 may include a single-modality scanner and/or a multi-modality scanner. The single-modality scanner may include, for example, an ultrasound scanner, an X-ray scanner, a computed tomography (CT) scanner, a magnetic resonance imaging (MRI) scanner, a sonographer, a positron emission tomography (PET) scanner, an optical coherence tomography (OCT) scanner, an ultrasound (US) scanner, an intravascular ultrasound (IVUS) scanner, a near-infrared spectroscopy (NIRS) scanner, a far infrared (FIR) scanner, etc., or any combination thereof. The multi-modality scanner may include, for example, an X-ray imaging-magnetic resonance imaging (X-ray-MRI) scanner, a positron emission tomography-X-ray imaging (PET-X-ray) scanner, a single photon emission computed tomography-nuclear magnetic resonance imaging (SPECT-MRI) scanner, positron emission tomography-computed tomography (PET-CT) scanner, a digital subtraction angiography-magnetic resonance imaging (DSA-MRI) scanner, etc. The scanners provided above are for illustrative purposes only and are not intended to limit the scope of the present disclosure. As used herein, the term "imaging modality" or "modality" broadly refers to an imaging method or technique that collects, generates, processes, and/or analyzes imaging information of a target object. In the embodiment of the present disclosure, the imaging device 110 may be a positron emission computed tomography (PET) device.

In some embodiments, the imaging device 110 may include modules and/or components for performing imaging and/or correlation analysis. In some embodiments, the imaging device 110 may include accessory components and imaging components. Among them, the accessory components may refer to various facilities designed to satisfy the needs of clinical diagnosis and treatment with the imaging components. For example, it may include mechanical equipment such as an examination bed, a diagnostic bed, a catheter bed, a photography bed, etc., various support components, suspension components, brake components, hold components, a wire grid, a filter plate, a shutter, etc. In some embodiments, the imaging components may take a variety of forms, for example, a digital imaging component may include a detector, a computer system, and an image processing software, etc.; any other imaging component may include a phosphor filter, a film cassette, an image intensifier, a video television, etc.

In some embodiments, the detector may include a plurality of detector modules, and each detector module may include a plurality of detector units. In some embodiments, each detector module may include a photosensitive module and a readout circuit. Among them, the photosensitive module may be configured to collect photon signals generated by radionuclides injected by the target object and convert the collected photon signals into electrical signals, and the readout circuit may be configured to read out electrical signals in the photosensitive module and convert the electrical signals into digitizing data, so that images may be generated. In some embodiments, the photosensitive module may include a crystal. A crystal is a structure in which a large amount of microscopic material units (atoms, ions, molecules, etc.) are arranged in an orderly manner, such as a crystalline Si (silicon), a crystalline CdTe, a crystalline GaAs, a crystalline HgI2, and a crystalline CdZnTe (CZT). In some embodiments, the photosensitive module may use scintillation crystals as a photosensitive substrate. The scintillation crystals refer to crystals that may convert the kinetic energy of high-energy at least one particle into light energy under the impact of high-energy at least one particle (e.g., X-rays, gamma at least one photon) to emit flashes. In some embodiments, the detector may include a semiconductor detector, a photovoltaic detector, etc., which are not limited to the present disclosure.

In some embodiments, data obtained by the imaging device 110 (e.g., scan data of a target object) may be transmitted to the processing device 140 for further analysis. Additionally or alternatively, data obtained by the imaging device 110 may be sent to an end device (e.g., the terminal(s) 130) for display and/or a storage device (e.g., the storage device 150) for storage.

The network 120 may include any suitable network capable of facilitating the exchange of information and/or data for the system 100. In some embodiments, at least one component of the system 100 (e.g., the imaging device 110, the terminal(s) 130, the processing device 140, the storage device 150) may exchange information and/or data with at least one other component in the system 100 via the network 120. For example, the processing device 140 may obtain background single events, at least one background coincidence event, and the like from detectors of the imaging device 110 via the network 120. The network 120 may include a cable network, a wireline network, a fiber-optic network, a telecommunications network, an intranet, a wireless local area network (WLAN), a metropolitan area network (MAN), a public telephone switched network (PSTN), a Bluetooth^{™} network, a ZigBee^{™} network, a near field communication (NFC) network, or the like, or any combination thereof. In some embodiments, the network 150 may include one or more network access points. For example, the network 150 may include wired and/or wireless network access points such as base stations and/or internet exchange points through which one or more components of the system 100 may be connected to the network 120 to exchange data and/or information.

The terminal(s)130 may communicate and/or connect with the imaging device 110, the processing device 140 and/or the storage device 150. For example, a user may interact with the imaging device 110 through the terminal(s) 130 to control one or more components of the imaging device 110. In some embodiments, the terminal(s) 130 may include a mobile device 131, a tablet computer 132, a laptop computer 133, etc., or any combination thereof. For example, the mobile device 131 may include a mobile control stick, a personal digital assistant (PDA), a smartphone, etc., or any combination thereof.

In some embodiments, the terminal(s) 130 may include an input device, an output device, and the like. The input device may select a keyboard input, a touch filter (e.g., with tactile or tactile feedback) input, a voice input, an eye-tracking input, a gesture-tracking input, a brain monitoring system input, an image input, a video input, or any other similar input mechanism. Input information received through the input device may be transmitted, e.g., via a bus, to the processing device 140 for further processing. Other types of input devices may include a cursor control device such as a mouse, a trackball, cursor direction keys, or the like. In some embodiments, an operator (e.g., a medical staff) may input an instruction to scan a target object, an instruction to detect the state of the imaging device 110, and the like through an input device. The output device may include a display, a speaker, a printer, etc., or any combination thereof. The output device may be configured to output an image of the target object scanned by the imaging device 110, and/or an image determined by the processing device 140, and the like. In some embodiments, the terminal(s) 130 may be part of the processing device 140.

The processing device 140 may process data and/or information obtained from the imaging device 110, the terminal(s) 130, the storage device 150, or other components of the system 100. For example, the processing device 140 may obtain a first background event, a second background event, a third background event, a fourth background event, etc. from the imaging device 110, and analyze and process them, so as to detect a state of the imaging device 110. In some embodiments, the processing device 140 may be a single server or a server group. The server group may be centralized or distributed. In some embodiments, the processing device 140 may be local or remote. For example, the processing device 140 may access information and/or data from the medical device 110, the storage device 150, and/or the terminal(s) 130 via the network 150. As another example, the processing device 140 may be directly connected to the medical device 110, the terminal(s) 130, and/or the storage device 150 to access information and/or data. In some embodiments, the processing device 140 may be implemented on a cloud platform. For example, the cloud platform may include a private cloud, a public cloud, a hybrid cloud, a community cloud, a distributed cloud, an inter-cloud, a multi-cloud, or the like, or a combination thereof.

In some embodiments, the processing device 140 may include one or more processors (e.g., a single-chip processor or a multi-chip processor). For example, the processing device 140 may include a central processing unit (CPU), an application-specific integrated circuit (ASIC), an application-specific instruction-set processor (ASIP), a graphics processing unit (GPU), a physics processing unit (PPU), a digital signal processor (DSP), a field-programmable gate array (FPGA), a programmable logic device (PLD), a controller, a microcontroller unit, a reduced instruction-set computer (RISC), a microprocessor, or the like, or any combination thereof. In some embodiments, the processing device 140 may be a part of the imaging device 110 or the terminal(s) 130. For example, the processing device 140 may be integrated within the imaging device 110 for detecting a state of the imaging device 110 based on a background single event or at least one background coincidence event.

The storage device 150 may store data, instructions, and/or any other information. For example, the storage device 150 may store the background events and related information thereof obtained by the imaging device 110, and the like. In some embodiments, storage device 150 may store data obtained from the imaging device 110, the terminal(s) 130, and/or the processing device 140. In some embodiments, the storage device 150 may store data and/or instructions used by the processing device 140 to perform or use to accomplish the example methods described herein. In some embodiments, the storage device 150 may include a mass storage, a removable storage, a volatile read-write memory, a read-only memory (ROM), or the like, or any combination thereof. In some embodiments, the storage device 150 may be implemented on a cloud platform.

In some embodiments, the storage device 150 may be connected to the network 120 to communicate with at least one other component in system 100 (e.g., the imaging device 110, terminal(s) 130, the processing device 140). At least one component in system 100 may access data stored in the storage device 150 via the network 120 (e.g., background single-event information, background coincident event information, single-event images, etc.). In some embodiments, the storage device 150 may be part of the processing device 140.

It should be noted that the above description is provided for illustrative purposes only and is not intended to limit the scope of the present disclosure. Numerous changes and modifications may be made to those of ordinary skill in the art under the guidance of the contents of the present disclosure. Features, structures, methods, and other features of the exemplary embodiments described herein may be combined in various ways to obtain additional and/or alternative exemplary embodiments. For example, the storage device 150 may be a data storage device including a cloud computing platform (e.g., a public cloud, a private cloud, a community, and a hybrid cloud, etc.). However, such changes and modifications do not depart from the scope of the present disclosure.

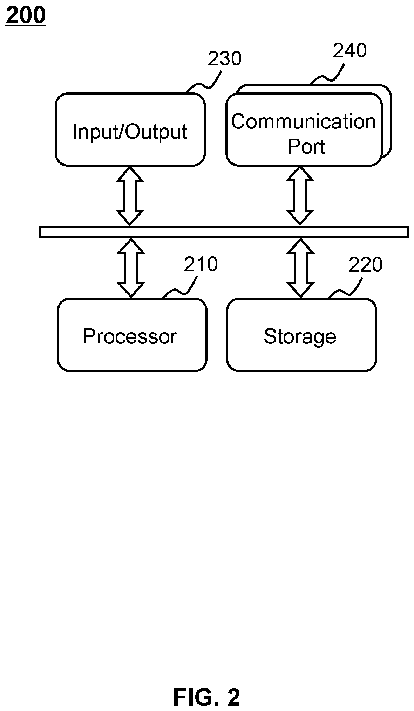

FIG. 2 is a schematic diagram of exemplary hardware and/or software of a computing device according to some embodiments of the present disclosure.

As shown in FIG. 2, the computing device 200 may include a processor 210, a storage 220, an input/output interface 230, and a communication port 240.

The processor 210 may execute computing instructions (program codes) and execute functions of the system 100 for state detection of the imaging device described herein. The computing instructions may include programs, objects, components, data structures, procedures, modules, and functions (the functions refer to specific functions described in the present disclosure). For example, the processor 210 may process information obtained from any component of the system 100 for background events. In some embodiments, the processor 210 may include a microcontroller, a microprocessor, a reduced instruction set computer (RISC), an application-specific integrated circuit (ASIC), an application-specific instruction set processor (ASIP), a central processing unit (CPU), a Graphics Processing Unit (GPU), a Physical Processing Unit (PPU), a Microcontroller Unit, a Digital Signal Processor (DSP), a Field Programmable Gate Array (FPGA), an Advanced RISC Machine (ARM), a Programmable Logic Device, and any circuit, processor, etc., or any combination thereof, that perform one or more functions. For illustration only, the computing device 200 in FIG. 2 only depicts one processor, but it should be noted that the computing device 200 in the present disclosure may also include a plurality of processors.

The storage 220 may store data/information obtained from any other component of system 100. In some embodiments, the storage device 130 may include a mass storage device, a removable storage device, a volatile read-and-write memory, a read-only memory (ROM), or the like, or any combination thereof. Exemplary mass storage devices may include a magnetic disk, an optical disk, a solid-state drive, etc. Exemplary removable storage devices may include a flash drive, a floppy disk, an optical disk, a memory card, a zip disk, a magnetic tape, etc. Exemplary volatile read-and-write memory may include a random access memory (RAM). Exemplary RAM may include a dynamic RAM (DRAM), a double date rate synchronous dynamic RAM (DDR SDRAM), a static RAM (SRAM), a thyristor RAM (T-RAM), and a zero-capacitor RAM (Z-RAM), etc. Exemplary ROM may include a mask ROM (MROM), a programmable ROM (PROM), an erasable programmable ROM (EPROM), an electrically erasable programmable ROM (EEPROM), a compact disk ROM (CD-ROM), and a digital versatile disk ROM, etc.

The input/output interface 230 may be configured to input or output signals, data, or information. In some embodiments, the input/output interface 230 may allow a user to communicate with components in the system 100 (e.g., the imaging device 110). In some embodiments, the input/output interface 230 may include an input device and an output device. The input device may include a keyboard, a mouse, a touch filter, a microphone, or the like, or a combination thereof. The output device may include a display device, a speaker, a printer, a projector, etc., or any combination thereof. The display device may include a display (e.g., a liquid crystal display (LCD), a light-emitting diode (LED)-based display, a flat panel display, a curved screen, a television device, a cathode ray tube (CRT), a touch screen), a speaker, a printer, or the like, or a combination thereof. The communication port 240 may connect to the network for data communication. The connection may be a wired connection, a wireless connection, or a combination of both. The wired connection may include an electrical cable, an optical cable, or a telephone wire, or the like, or any combination thereof. The wireless connection may include, for example, a Bluetooth^{™} link, a Wi-Fi^{™} link, a WiMax^{™} link, a WLAN link, a ZigBeeTM link, a mobile network link (e.g., 3G, 4G, 5G), or the like, or a combination thereof. In some embodiments, the communication port 240 may be a standardized port such as RS232, RS485, or the like. In some embodiments, the communication port 240 may be a specially designed port. For example, the communication port 240 may be designed based on the digital imaging and communications in medicine (DICOM) protocol.

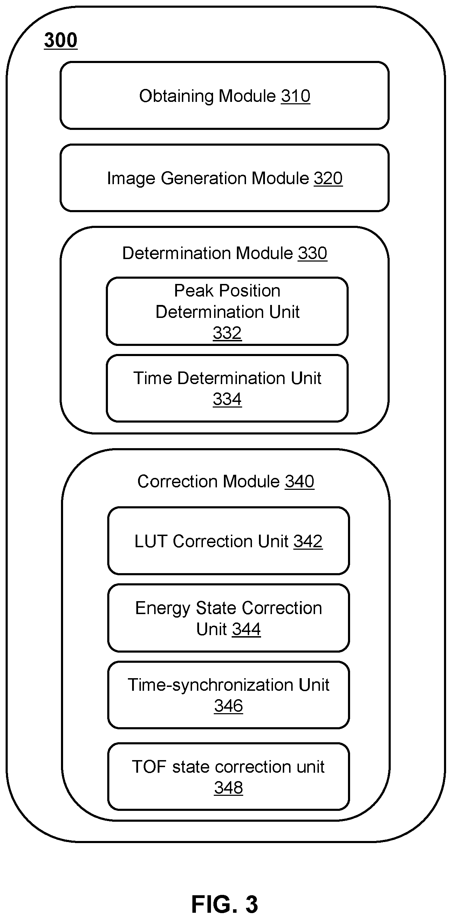

FIG. 3 is an exemplary block diagram of a state correction device of an imaging device according to some embodiments of the present disclosure.

As shown in FIG. 3, in some embodiments, the state correction device 300 of the imaging device may include an obtaining module 310, an image generation module 320, a determination module 330, and a correction module 340.

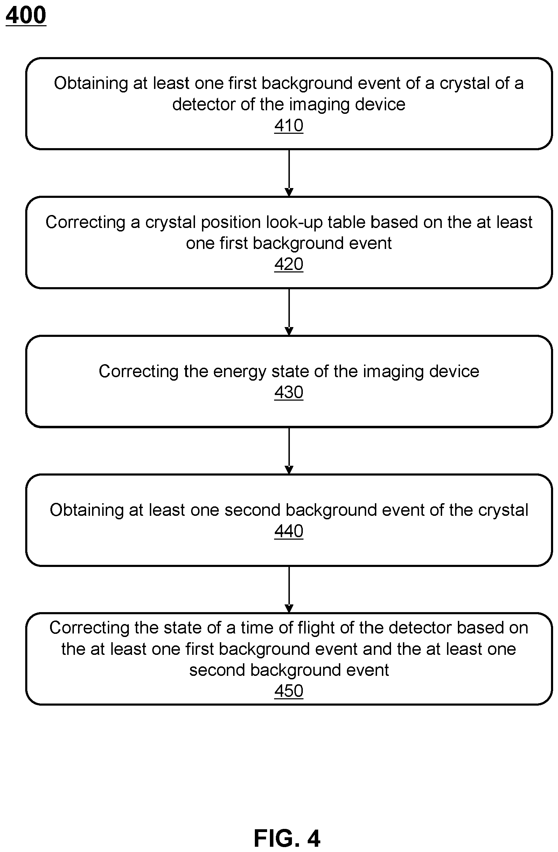

The obtaining module 310 may be configured to obtain data and/or information. For example, the obtaining module 310 may be configured to obtain information about background events received by the detector of the imaging device 110 related to inherent radiating particle(s) of crystal(s) of the detector. In some embodiments, the obtaining module 310 may be configured to obtain a background single event and/or at least one background coincidence event of the crystal(s) of the detector of the imaging device 110 (e.g., the first background event, the second background event, the third background event, the fourth background event, a fifth background event, and a sixth background event described in FIG. 4). In some embodiments, the obtaining module 310 may be configured to obtain a background event (e.g., the first background event) of the crystal(s) of the detector of the imaging device 110 in response to an abnormal state of the crystal position look-up table of the imaging device 110. In some embodiments, the obtaining module 310 may be configured to obtain at least one background event (e.g., the second background event or the third background event) of the crystal(s) of the detector of the imaging device 110 in response to corrections made to the crystal position look-up table of the imaging device 110.

The image generation module 320 may be configured to generate images related to the background events. For example, the image generation module 320 may be configured to generate a single-event image based on a background event (e.g., the first background event). In some embodiments, the image generation module 320 may be configured to generate an energy spectrogram based on energy information of a background event (e.g., the first background event, or the third background event). In some embodiments, the image generation module 320 may be configured to generate an event time chart based on information about a background event (e.g., the first background event and/or the second background event, or the fourth background event). In some embodiments, the image generation module 320 may also be configured to generate other related images, for example, an image of the target object, etc., which is not limited to the present disclosure.

The determination module 330 may be configured to determine a time of flight (TOF). In some embodiments, the determination module 330 may be configured to calculate a measured time of flight and a theoretical time of flight based on information of at least one background coincidence event (e.g., at least one background coincidence event filtered from the first background event and the at least one second background event, or the fourth background event in FIG. 4). In some embodiments, the determination module 330 may be further configured to determine at least one peak position in the energy spectrogram based on the energy spectrogram. In some embodiments, the determination module 330 may also be configured to determine a corresponding relationship between time-to-digital converter (TDC, Time-to-Digital Converter) values and times.

In some embodiments, the determination module 330 may further include a peak position determination unit 332 and a time determination unit 334. The peak position determination unit 332 may be configured to calculate the peak position of the spectrogram. The time determination unit 334 may be configured to calculate event-related time information, e.g., the measured time of flight, the theoretical time of flight, and the like.

The correction module 340 may be configured to correct the state of the imaging device. The states of an imaging device may refer to states of a software and/or a hardware of the imaging device, as well as the accuracy states of system data, and the like. The states of the imaging device that the correction module 340 may use to correct may include: a state of the crystal position look-up table, an energy scale state, an energy correction state, a time synchronization state between detector modules, a state of time of flight, a TDC scale state, a system failure, etc., or any combination thereof. For example, the correction module 340 may be configured to correct the crystal position look-up table of the imaging device based on information of a background event (e.g., the first background event). As another example, the correction module 340 may be configured to correct an energy state of the imaging device based on the information of the at least one background event (e.g., the first background event or the third background event). As another example, the correction module 340 may be configured to perform a time correction on the detector of the imaging device based on the information of the at least one background coincidence event (e.g., the at least one background coincidence event obtained based on the first background event and the at least one second background event).

In some embodiments, the correction module 340 may further include a LUT correction unit 342, an energy state correction unit 344, a time-synchronization unit 346, and a TOF state correction unit 348.

The LUT correction unit 342 may be configured to correct the crystal position look-up table of the imaging device. In some embodiments, the LUT correction unit 342 may determine (e.g., before correcting the crystal position look-up table) whether the crystal position look-up table state is abnormal. Whether the state of the crystal position look-up table of the imaging device is abnormal may refer to whether a response position of each crystal(s) of the detector with respect to each event obtained by calculation is consistent with an actual position of the crystal for the event detected by the detector. In some embodiments, in response to an abnormal crystal position look-up table state, the LUT correction unit 342 may correct the crystal position look-up table based on the information of the at least one background event (e.g., the first background event).

The energy state correction unit 344 may be configured to correct the energy state of the imaging device. In some embodiments, the energy state correction unit 344 may determine (e.g., before correcting the energy state) whether the energy state is abnormal. The energy state of an imaging device may refer to a matching state of energy values obtained when each of the detector channels receives at least one particle of the same energy. Whether the energy state is abnormal may refer to whether energy values obtained by at least one particle of the same energy received by each detector channel are the same. In some embodiments, if the energy state of the imaging device is abnormal, an energy scale curve may shift accordingly. The energy scale curve maps ADC values that refer to particle energies to actual energies. The accuracy of the energy scale curve may be related to the image resolution of the PET device. As used herein, the ADC (analog-to-digital converter) refers to a device that converts a continuously changing analog signal into a discrete digital signal; the ADC value refers to a sampling value of the analog-to-digital converter. In some embodiments, the energy state correction unit 344 may correct the energy state based on information of a background event (e.g., the first background event or the third background event). For example, the energy state correction unit 344 may determine at least two energy peak values related to a nuclide decay of the crystal and ADC values corresponding to at least two energy peak values based on information of the at least one third background event; determine an energy scale curve of the imaging device based on the at least two energy peak values and ADC values corresponding to the at least two energy peak values.

The time-synchronization unit 346 may be configured for a time synchronization of each detector module of the detector of the imaging device. In some embodiments, the time-synchronization unit 346 may determine (e.g., before or at the same time as correcting the time of each detector module) whether the time of each detector module is synchronized. In some embodiments, in response to the time of each detector module being out of synchronization, the time-synchronization unit 346 may perform the time-synchronization of each detector module based on the information of the at least one background coincidence event(s). For example, the time-synchronization unit 346 may perform the time-synchronization of each detector module of the detector based on the measured time of flight and the theoretical time of flight.

The TOF state correction unit 348 may be configured to correct a state of time of flight of the imaging device. In some embodiments, the TOF state correction unit 348 may determine (e.g., before correcting the state of a time of flight) whether the state of a time of flight is abnormal. The state of a time of flight may refer to a state of a time at which the particle was received by the detector. Whether the state of a time of flight is abnormal may refer to whether the time of flight drifts. In some embodiments, in response to a state of a time of flight anomaly (e.g., a difference between the measured time of flight of the at least one background coincidence event and the theoretical time of flight exceeds a threshold), the TOF state correction unit 348 may detect detection based on the information of the at least one background coincidence event. The time of flight of the aircraft is corrected. In some embodiments, the TOF state correction unit 348 may also determine the TDC scale curve of the imaging device based on the corresponding relationship between the TDC value and time.

It should be noted that the above description of the state correction device 300 of the imaging device and its modules is only for the convenience of description, and does not limit the present disclosure to the scope of the illustrated embodiments. It can be understood that for those skilled in the art, after understanding the principle of the system, various modules may be combined arbitrarily, or a subsystem may be formed to connect with other modules without departing from the principle. For example, in some embodiments, different modules in the obtaining module 310, the image generation module 320, the determination module 330, and the correction module 340 (e.g., the processing device 140) disclosed in FIG. 3 may also be implemented as one module that realizes functions of two or more of the above modules. As another example, each module may have its own storage module. As another example, each module may share one storage module. Such deformations are all within the protection scope of the present disclosure.

FIG. 4 is an exemplary flowchart of a state detection method of an imaging device according to some embodiments of the present disclosure.

In some embodiments, the method 400 for state detection of an imaging device (e.g., a PET device) may be performed by the system 100 for state detection (e.g., the processing device 140) or the state correction device 300 of the imaging device. For example, the method 400 for state detection of an imaging device may be stored in a storage device (e.g., the storage device 150) in a form of a program or instructions, and when the system 100 for state detection (e.g., the processing device 140) executes the program or instructions, the imaging device may be implemented the method 400 for state detection. The schematic diagram of the operation of the method 400 for state detection presented below is illustrative. In some embodiments, the process may be accomplished using one or more additional operations not described and/or one or more operations not discussed. Additionally, the order in which the operations of the method 400 for state detection are shown in FIG. 4 and described below is not intended to be limiting.

In 410, the processing device 140 may obtain at least one first background event of a crystal of a detector of the imaging device. In some embodiments, operation 410 may be performed by the obtaining module 310.

In some embodiments, the detector of the imaging device may comprise a plurality of detector crystals, and a pair of γ photons produced by positron annihilation may be received by crystals of two different detectors. When at least one γ photon are incident on the detector of the imaging device, a large number of at least one photon may be generated on the crystal, and the at least one photon may be received by the detector and converted into electrical signals. By weighting the signals generated by a plurality of detectors, an action position of the at least one γ photon may be calculated. However, due to an actual design of the detector and problems of an algorithm itself, the image may have pincushion or barrel distortion, and an actual calculated position may not be a real action position of the at least one γ photon. Therefore, the detector may need to be irradiated with a pan-field source, and an obtained pan-field image may be segmented to obtain a response position of each crystal, which may be used as a look-up table (LUT) of the crystal position of the detector. In the actual collection, the crystal that interacts with the at least one γ photon may be determined based on the calculated action position of the at least one γ photon and the crystal position look-up table, and the actual physical position of the crystal in the system may be used as the action position of the at least one γ photon, so as to determine an incident position of the γ photon. Therefore, the accuracy of the crystal position look-up table may be related to the accuracy of the detection of the incident position of the at least one γ photon, and the accuracy of the detection of the incident position of the at least one γ photon may ensure an image resolution of the imaging device. However, the crystal position look-up table may deviate due to, for example, unclear pan-field images, so it may need to be corrected. In some embodiments, the crystal(s) of the detector of the imaging device may include a scintillation crystal such as a sodium iodide (Nal) crystal, a bismuth germanate (BGO) crystal, a lutetium silicate (LSO) crystal, a lutetium yttrium silicate (LYSO) crystal. In the embodiments of the present disclosure, a detector of the lutetium yttrium silicate (LYSO) scintillation crystal may be mainly used as an example for description.

In some embodiments, the processing device 140 may periodically detect a state of the crystal position look-up table of the imaging device 110 based on a preset time interval (e.g., one hour, one day, one week, five days, etc.). In some embodiments, the processing device 140 may detect the state of the crystal position look-up table of imaging device 110 in real-time (e.g., when the imaging device is idle). For example, the processing device 140 may detect whether the state of the crystal position look-up table of the imaging device 110 is abnormal before the imaging device 110 scans the target object.

The background events described herein (e.g., the first background event, the second background event, the third background event, the fourth background event, etc.) may be related to inherent radiating particle(s) of the crystal(s) of the detector of the imaging device. Taking the lutetium yttrium silicate (LYSO) scintillation crystal as an example of the crystal(s) of the detector, due to the presence of Lu176 in LYSO, the crystal has an inherent radiation phenomenon, which may also be referred to as a background radiation phenomenon. When Lu 176 decays, at least one β particle and at least one γ photon may be generated. The at least one generated β particle may have a short range, and the energy of the generated at least one β particle may be mainly deposited in the decaying crystal; the at least one generated γ photon may have high penetrating power, and the at least one generated γ photon may not only be detected in a crystal where decay occurs, but also may escape and be detected by another crystal. An event in which the detector receives the at least one β particle and/or at least one γ photon may be referred to as a background event. In some embodiments, the at least one background event may include at least one background single event or at least one background coincidence event. As an example only, a particle generated by a decay of Lu176 may be detected only in the same crystal, and an event that the detector corresponding to the crystal receives the particle may be referred to as the background single event. If a γ photon generated by the decay escaped and is detected by another crystal, a line connecting a detector corresponding to a crystal receiving the β particle and a detector corresponding to the crystal receiving the γ photon may be referred to as a response line; and an event that the detectors receive the β particle and the γ photon may be referred to as a background coincidence event. In some embodiments, data related to the receiving of the β particle and/or the γ photon by the detector(s) may be referred to as background event information.

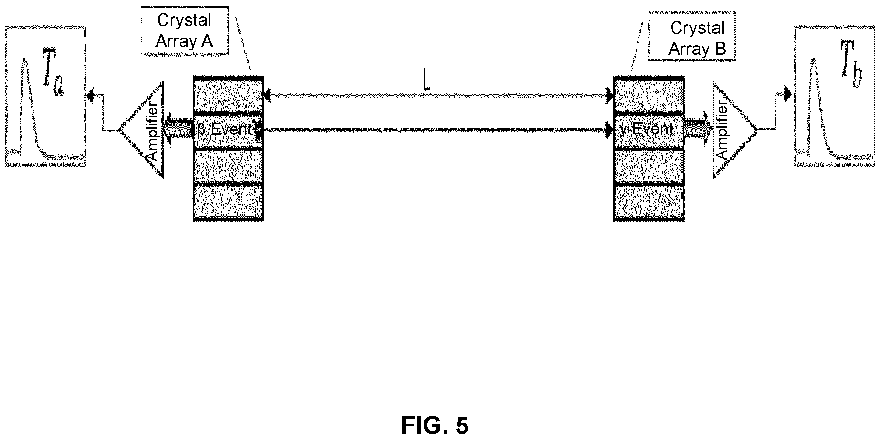

As shown in FIG. 5, a detector of the imaging device may include a crystal array A and a crystal array B. A β event may be generated in the crystal array A, and a corresponding γ event may be received in the corresponding crystal array B. The crystal array A may be coupled to an optical sensor and a front-end circuit, and the front-end circuit may include an amplifier for recording β event information; the crystal array B may be coupled to an optical sensor and a front-end circuit, and the front-end circuit may include an amplifier for recording γ event information. The information of a background coincidence event may be: time information Ta of a time when the crystal array A receives the β event and a position of a crystal that receives the β event in the crystal array A; time information Tb of a time when the crystal array B receives the γ event and a position of a crystal that receives the γ event in the crystal array B. The position may be a coordinate position of a corresponding crystal obtained by directly establishing a coordinate system with the entire detector.

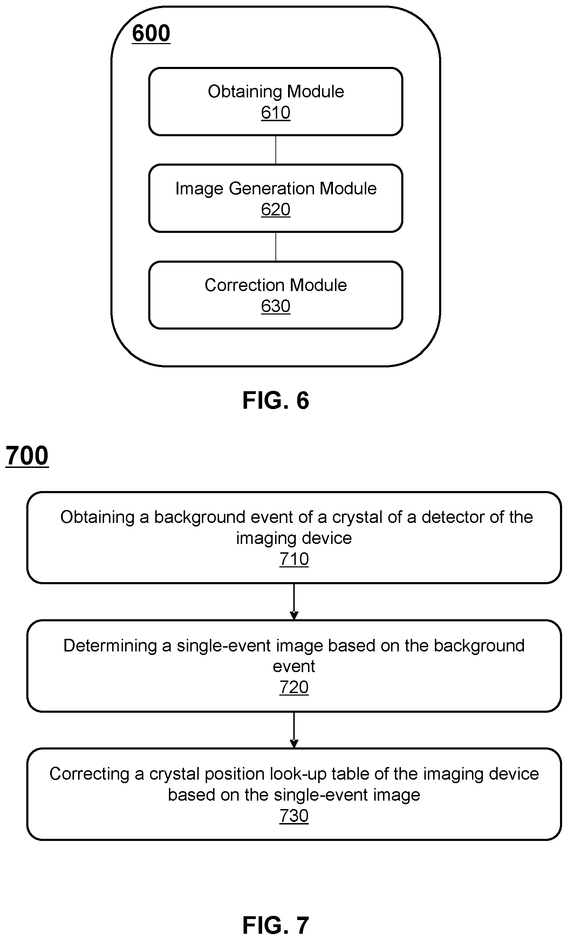

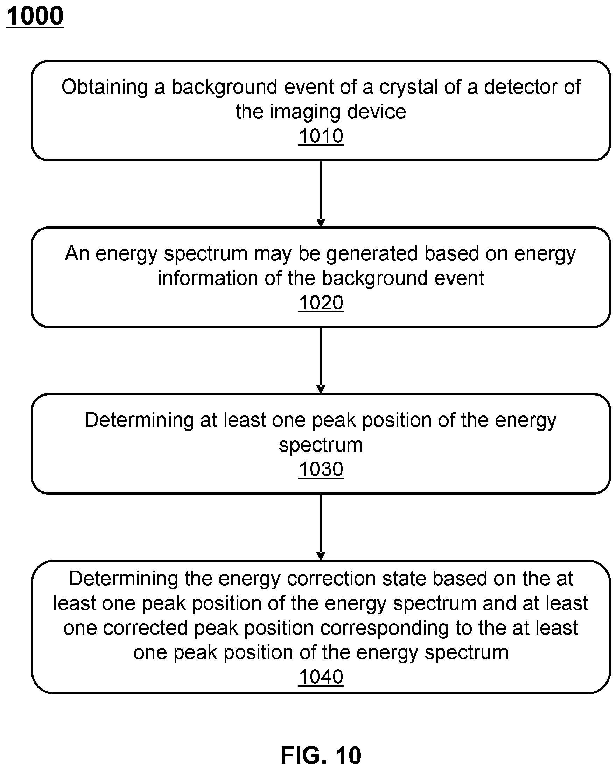

In some embodiments, the processing device 140 may obtain at least one first background event of at least one inherent radiating particle of at least one crystal received by the detector(s) based on a preset energy window. The first background event may be a background single event. In some embodiments, a value range of the energy window may be within a clinical threshold value range of the energy window of the imaging device. In some embodiments, the processing device 140 may obtain the at least one first background event of the inherent radiating particle of the crystal received by the detector based on a preset time window. For more contents about the energy window, please refer to the related description in FIG. 7, and For more contents about the time window, please refer to the related description in FIG. 10, which will not be repeated here.

In 420, the processing device 140 may correct a crystal position look-up table based on the at least one first background event. In some embodiments, operation 420 may be performed by the correction module 340 (e.g., the LUT correction unit 342).

In some embodiments, the processing device 140 may determine a single-event image based on the at least one first background event; and/or correct the crystal position look-up table based on the single-event image. For more details about the corrected crystal position look-up table, please refer to other parts of the present disclosure, for example, FIG. 7 and its related descriptions, which will not be repeated here.

In 430, the processing device 140 may correct the energy state of the imaging device. In some embodiments, operation 430 may be performed by the correction module 340 (e.g., the energy state correction unit 344).

A digital processing part of the detector may convert the at least one incident γ photon into electrical signals to obtain information such as the energy, the position, and time of the at least one γ photon. The position of at least one response line where the at least one background event is located may be determined through an energy coincidence calculation. The digital processing part of the detector may further obtain distributions of positron nuclides in the target object by a 2D or 3D tomographic reconstruction algorithm, and thus the physiological and/or biochemical processes in the target object may be observed *in vitro.* When performing the energy coincidence calculation, an energy calculation of each detector module may be conducted based on data of the detector module. Therefore, for a certain detector, the energy calculations of various detection modules may be independent. However, due to differences that may exist between the plurality of crystals and photoelectric conversion devices used in the detector of the imaging device, as well as between the various circuits, after using the same method to calculate the energy of the γ photons of the same energy received by the crystals, an energy value corresponding to a counted peak value detected by each crystal may exhibit a certain offset from an energy value corresponding to a theoretical counted peak value. Therefore, the energy state of the imaging device may need to be corrected.

In some embodiments, the processing device 140 may periodically perform an energy state correction on the imaging device 110 based on a preset time interval (e.g., 5 hours, 10 hours, 3 days, 5 days, 1 month, etc.). In some embodiments, the processing device 140 may perform the energy state correction on the imaging device when the imaging device is in normal use or when the imaging device is idle. In some embodiments, the processing device 140 may perform the energy state correction on a detector module of the imaging device after the detector module is replaced (e.g., after one or more detection modules of the imaging device have failed and been replaced). In some embodiments, the processing device 140 may perform the energy state correction on the imaging device after performing a correction on the crystal position look-up table of the imaging device. In some embodiments, the processing device 140 may perform the energy state correction on the imaging device in response to an abnormal energy state of the imaging device.

In some embodiments, the processing device 140 may correct the energy state of the imaging device based on the at least one first background event. In some embodiments, the processing device 140 may obtain the at least one third background event of the crystal(s) of the detector of the imaging device, and correct the energy state of the imaging device based on the at least one third background event. As used herein, the third background event may be related to the inherent radiating particle(s) of the crystal(s) of the detector of the imaging device, and the third background event may include at least one background single event or at least one background coincidence event of the inherent radiating particle of the crystal received by the detector of the imaging device. For example, if the event-obtaining mode is a single event mode (correspondingly, the first background event is the background single event), the processing device 140 may first correct the crystal position look-up table of the detector of the imaging device 110 based on the background single event in operation 420, and then correct the energy state of the imaging device 110 based on the background single event in operation 430. As used herein, the single event mode may refer to an event-obtaining mode corresponding to an obtaining of the background single event by the detector of the imaging device. For another example, the processing device 140 may obtain the at least one third background event of the crystal after correcting the crystal position look-up table of the detector of the imaging device 110 based on the at least one first background event, and correct the energy state of the imaging device 110 based on the at least one third background event.

In some embodiments, the processing device 140 may determine whether the energy state of the imaging device 110 is abnormal. Specifically, the processing device 140 may generate an energy spectrogram based on the energy information of the at least one first background event or the at least one third background event, and determine at least one peak position in the energy spectrogram; determine the energy correction state of the imaging device based on the at least one peak position, and at least one corrected peak position corresponding to the at least one peak position; correct the energy state of the imaging device in response to an abnormality of the energy correction state. In some embodiments, the processing device 140 may directly correct the energy state of the imaging device 110 without judging whether the energy state of the imaging device 110 is abnormal. In some embodiments, the third background event may be a background single event, and the processing device 140 may generate the energy spectrogram based on energy information of the background single event. In some embodiments, the processing device 140 may filter particle energy information based on a preset time window and/or a preset energy window based on an arrival time of at least one particle of the at least one third background event received by the detector of the imaging device 110 in a coincidence event mode (i.e., the third background event is a background coincidence event); and generate the energy spectrum based on filtered particle energy information. In some embodiments, the energy spectrogram may include at least one of a peak value of a full energy peak or a peak value of a single energy peak. The coincidence event mode may refer to a mode in which the at least one background coincidence event is obtained through coincidence processing after the detector of the imaging device receives the at least one background event. For more details about correcting the energy state, please refer to other parts of the present disclosure, for example, FIGs. 10-12 and their related descriptions, which will not be repeated here.

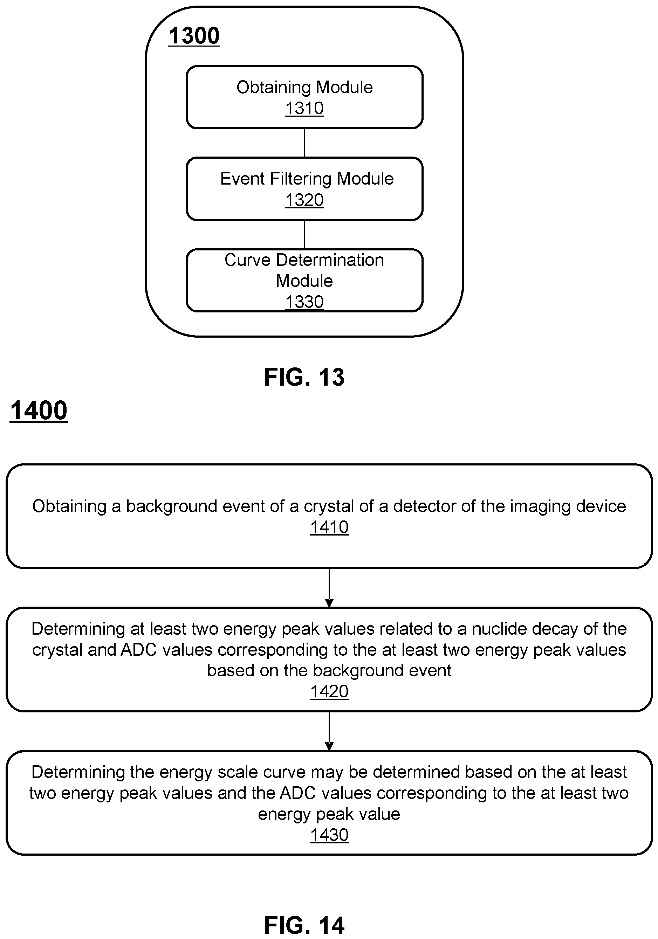

In some embodiments, the processing device 140 may determine, based on the at least one first background event or the at least one third background event, at least two energy peak values associated with a nuclide decay of the crystal and at least two energy peak values associated with the at least two ADC values corresponding to the at least two energy peak values; and determine an energy scale curve of the imaging device based on the at least two energy peak values and the ADC values corresponding to the at least two energy peak values. For more contents related to determining the energy scale curve, reference may be made to other parts of the present disclosure, for example, FIG. 14 and related descriptions, which will not be repeated here.

In 440, the processing device 140 may obtain at least one second background event of the crystal. In some embodiments, the operation 440 may be performed by the obtaining module 310.

The at least one second background event may be related to the inherent radiating particle of the crystal, and the at least one second background event may be a background single event. In some embodiments, the processing device 140 may obtain the at least one second background event after completing the energy state correction of the imaging device, or obtain the at least one second background event before the energy state correction or the correction of the crystal position look-up table, which are not limited to the present disclosure. In some embodiments, the processing device 140 may obtain the at least one second background event of the inherent radiating particle of the crystal received by the detector based on a preset energy window. In some embodiments, the value range of the energy window may be within the clinical threshold value range of the energy window of the imaging device. In some embodiments, the processing device 140 may obtain the at least one second background event of the inherent radiating particle of the crystal received by the detector based on a preset time window. In some embodiments, the range of the time window is within the range of a clinical time window threshold value of the imaging device.

In 450, the processing device 140 may correct the state of a time of flight of the detector based on the at least one first background event and the at least one second background event. In some embodiments, the operation 450 may be performed by the correction module 340 (e.g., the TOF state correction unit 348).

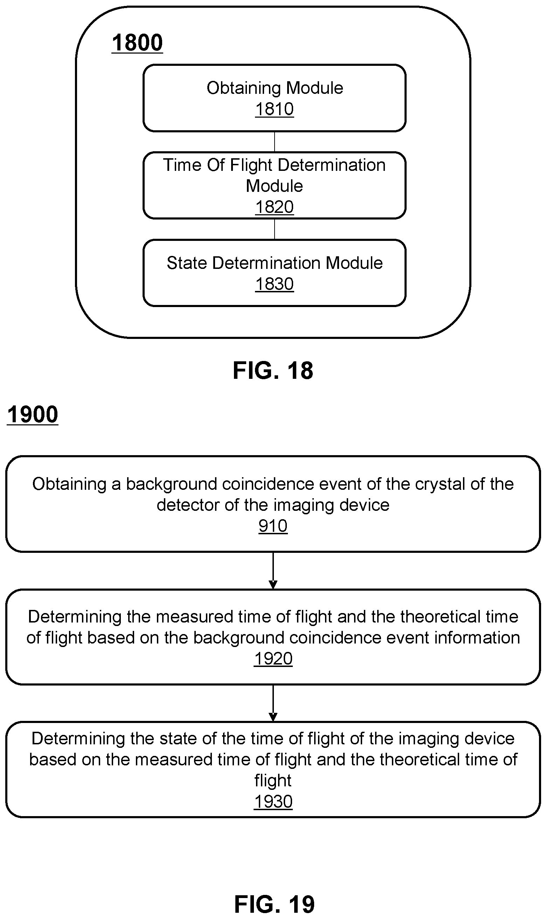

In some embodiments, the processing device 140 may determine the measured time of flight based on the at least one first background event and the at least one second background event, and a state of the time of flight of the imaging device may be reflected by the measured time of flight. The time of flight of the detector of the imaging device may be corrected. Specifically, the processing device 140 may obtain the at least one background coincidence event by performing a coincidence processing operation on the at least one first background event and the at least one second background event, and determine the measured time of flight and the theoretical time of flight based on the information of the at least one background coincidence event; and the time of flight of the detector may be corrected based on the information of the at least one background coincidence event. In some embodiments, the information of the background event may include a time of arrival of the at least one particle in the background event reaching the detector of the imaging device and a corresponding crystal position. In some embodiments, the processing device 140 may determine whether the state of a time of flight is abnormal before correcting the state of a time of flight. For more contents about the correction of the time of flight, please refer to other parts of the present disclosure, for example, FIG. 19 and its related description, which will not be repeated here.

In some embodiments, the processing device 140 may generate an event time chart based on the at least one first background event and the at least one second background event; determine a corresponding relationship between TDC values and time based on the event time chart; determine a TDC scale curve of the imaging device based on the corresponding relationship. In some embodiments, the processing device 140 may obtain the fourth background event of the crystal, and determine the TDC scale curve of the imaging device based on the fourth background event. The fourth background event may be related to the inherent radiating particle of the crystal. The fourth background event may include a background single event or at least one background coincidence event. In some embodiments, the processing device 140 may generate an event time chart based on the fourth background event; determine a corresponding relationship between the TDC values and time based on the event time chart; and determine the TDC scale curve of the imaging device based on the corresponding relationship.

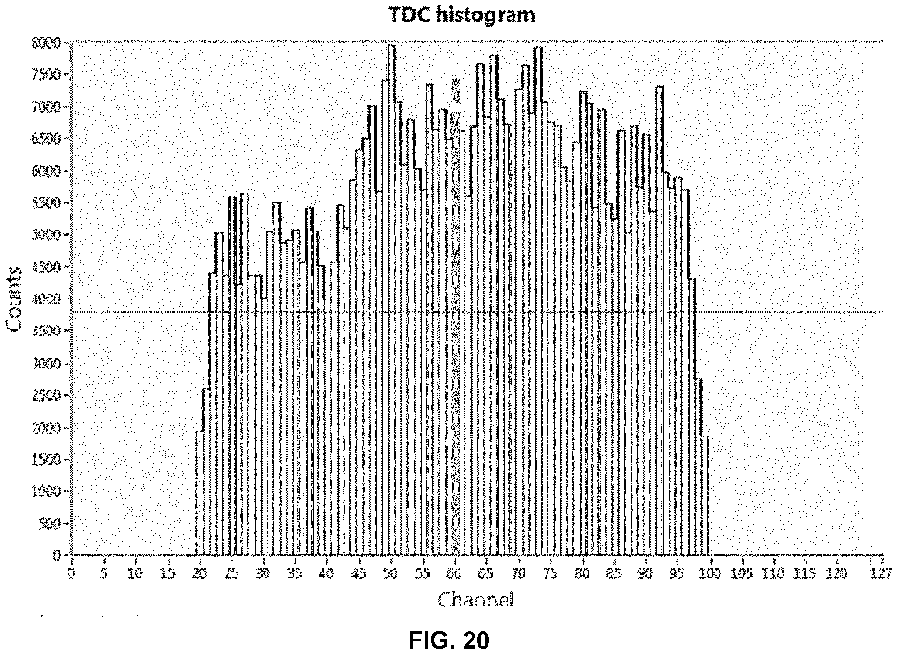

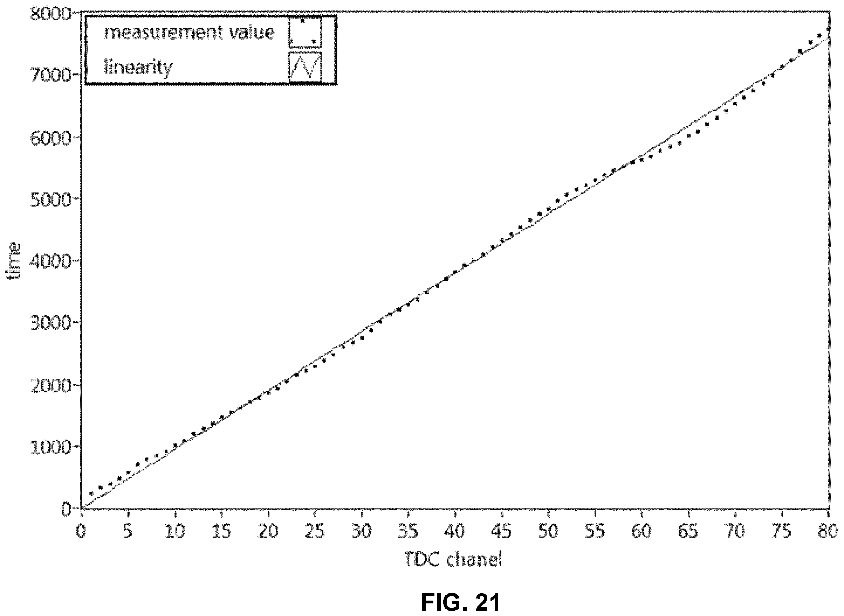

Merely as an example, the processing device 140 may determine a count Nᵢ of the at least one background event corresponding to each TDC value (i.e., event time chart) based on the background single event or the at least one background coincidence event (e.g., the fourth background event) of the inherent radiating particle(s) of the crystal(s) of the detector received by the detector. Herein i represents a TDC value, and i = 0, 1, 2, ... Specifically, after the detector of the imaging device receives the at least one background event, an arrival time of the at least one particle in the at least one background event to the detector may be recorded, and the arrival time may be converted into a corresponding electrical signal (i.e., a count of channels). The event time chart may be generate based on the electrical signal. In some embodiments, the greater a total count of collected background events, the smaller an error, and the higher an accuracy of the obtained TDC scale curve. For example, in order to reduce a statistical error, an average value of Nᵢs corresponding to the TDC values may be greater than 1000. Further, the processing device 140 may determine a reference TDC value and a reference time corresponding to the reference TDC value based on the event time chart. For example, as shown in FIG. 20, the processing device 140 may convert the TDC value 60 (that is, a channel value of a position corresponding to the vertical dotted line in FIG. 20) that may make the total count of background events corresponding to the TDC values on the left and right sides of the event time chart substantially equal into the reference TDC value, and denote the reference TDC value as i₀. Then, let a time corresponding to the reference TDC value be T/2 as the reference time, where T represents a clock period of the TDC values. Further, a corresponding relationship between TDC values on the left side of the reference TDC value and times in the event time chart may be determined through performing a recursive calculation by a mathematical formula: T_{I-1} = T_{I} -N_{I} * (T/ΣN_{I)}, where I = i₀, i₀ - 1, i₀ - 2, ..., iₛₜₐᵣₜ. A corresponding relationship between TDC values on the right side of the reference TDC value and times in the event time chart may be determined through performing a recursive calculation by a mathematical formula: T_{I+1} = T_{I} + NI * (T/ΣN_{I)}, where, I = i_{0,} i₀ + 1, i₀ + 2, ..., i_{end}, where T_{I = i0} = T/2. The processing device 140 may obtain a corresponding relationship between the TDC values and time T(i), i = 0, 1, 2, ..., based on the corresponding relationship between the TDC values and time on the left side of the reference TDC value and the corresponding relationship between the TDC values and time on the right side of the reference TDC value, and then obtain the TDC scale curve (as shown in FIG. 21). It can be understood that the above reference time position is only used as an example, in some alternative embodiments, other TDC values may be selected as the reference TDC value (for example, a TDC value in the event time chart with a TDC value of 0), so the reference time may be any time value in [0, T], which is not limited to the present disclosure. In some embodiments, a form of the TDC scale curve may include a form of a curve, a functional relationship, a look-up table, etc., which is not limited to the present disclosure. In some embodiments, after obtaining the TDC scale curve, the processing device 140 may determine a time corresponding to any TDC value on the TDC scale curve based on the TDC scale curve. In some embodiments, the processing device 140 may determine a time corresponding to any TDC value on the TDC scale curve based on the TDC scale curve, and perform a TOF correction or a time synchronization correction based on the determined time.

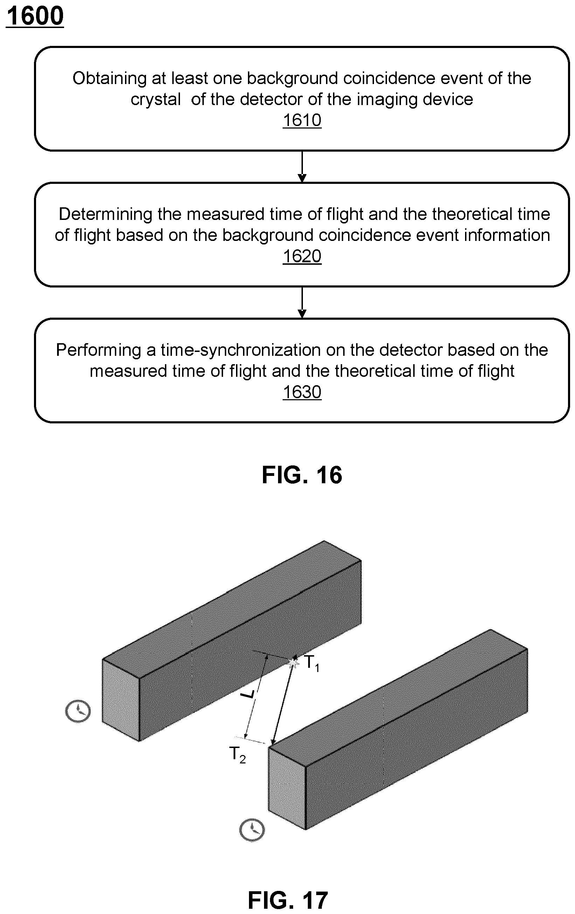

In some embodiments, the processing device 140 may determine the measured time of flight and the theoretical time of flight based on the at least one first background event and the at least one second background event; perform a time-synchronization on the detector modules of the detector based on the measured time of flight and the theoretical time of flight. In some embodiments, the processing device 140 may perform a time-synchronization on the detectors of the imaging device based on the fourth background event. For example, the fourth background event may include at least one background coincidence event of the inherent radiating particle of the crystal received by the detectors of the imaging device 110, and the processing device 140 may determine the measured time of flight and the theoretical time of flight based on the information of the at least one background coincidence event; time synchronize the detector modules in response to a difference between the measured time of flight and the theoretical time of flight exceeding a threshold value. In some embodiments, the processing device 140 may determine whether the time of the detector modules is synchronized before performing a time synchronization on the detector modules. For more contents about time synchronization, please refer to other parts of the present disclosure, for example, FIGs. 16-17 and related descriptions, which will not be repeated here.

In some embodiments, the processing device 140 may perform a correction on the crystal position look-up table and the energy state of the imaging device 110 based on the single background event (e.g., the at least one first background event or the second background event) obtained in the same round of acquisition. In some embodiments, the processing device 140 may detect and/or correct the energy correction state of the imaging device 110 and determine the energy scale curve based on the background single event or the background coincidence event (for example, the third background event) obtained in the same round of acquisition. In some embodiments, the processing device 140 may correct the state of a time of flight of the imaging device 110, perform the time-synchronization and/or determine the TDC scale curve based on the at least one background coincidence event (for example, the at least one background coincidence event, the third background event, or the fourth background event obtained from the at least one first background event and the at least one second background event) collected in the same round of acquisition. In some embodiments, the processing device 140 may determine the TDC scale curve of the imaging device 110 based on a background single event or at least one background coincidence event (for example, the third background event or the fourth background event).

In some embodiments, in response to that the crystal position look-up table of the imaging device 110 is corrected, the processing device 140 may perform the energy state correction on the imaging device, time synchronization on the detector modules, and/or the state of a TOF correction, etc. In some embodiments, the processing device 140 may perform time synchronization on the detector modules and state of a TOF correction on the imaging device in response to the energy state of the imaging device 110 being corrected. In some embodiments, any of energy state detection and/or correction, energy scale curve determination, time synchronization of the detector modules, state of a TOF correction, the determination of the TDC scale curve may be performed independently (i.e., the system 100 may collect the at least one background event separately for each state detection or correction, and different state detection or correction may not share the same background event data).

It should be noted that the above description of the process 400 is only for example and illustration, and does not limit the scope of application of the present disclosure. Various modifications and changes to the process 400 may be made to process 400 under the guidance of the present disclosure to those skilled in the art. For example, the processing device 140 may obtain the at least one first background event in response to an abnormal energy state of the imaging device 110, and correct the energy state of the imaging device 110 based on the at least one first background event. As another example, the processing device 140 may obtain the at least one first background event and the at least one second background event in response to the abnormal state of a time of flight of the imaging device 110, and correct the state of a time of flight of the imaging device 110 based on the at least one first background event and the at least one second background event. However, such corrections and changes are still within the scope of the present disclosure.

Ideally, a corresponding relationship between a physical position of a crystal array of the detector receiving annihilation effect of at least one γ photon and a position decoded to the image may be linear. However, due to the Compton scattering effect in the encoding process, non-uniform physical material of each crystal, and a nonlinear response of related electronic components, the corresponding relationship between the two may be nonlinear. The accuracy of the detection of the incident position of the gamma photon may guarantee the image resolution of the imaging device (e.g., a PET device). Therefore, in order to find the correspondence between the crystal and the image, a crystal position look-up table may be established, and the exact receiving position of the gamma photon may be determined through the look-up table.

LYSO scintillation crystals may be configured as scintillation crystals for the detector of the imaging device due to a high light output, a fast luminescence decay, a high effective atomic number, a high density, stable physicochemical properties, and a high detection efficiency of γ-rays of the LYSO scintillation crystals. Lu 176 existing in LYSO scintillation crystals may release at least one γ photon with energies of 88 keV, 202 keV, and 307 keV and at least one β particle with random energies ranging from 0 keV to 597 keV during a decay process.

The embodiments of the present disclosure provide a process for correcting the crystal position look-up table, which may be used to collect background events in a fixed energy range by using a background inherent radiation phenomenon of the crystal(s) of the detector under a condition of no radiation source for the detector module formed by coupling a crystal array with a light sensor detector with a certain regular arrangement to form a single-event image, and correct the crystal position look-up table through the single-event image.

FIG. 6 is an exemplary block diagram of a crystal position look-up table correction device according to some embodiments of the present application.

As shown in FIG. 6, the crystal position look-up table correction device 600 may include an obtaining module 610, an image generation module 620, and a correction module 630. In some embodiments, the obtaining module 610 and the obtaining module 310 may be modules of the same structure and/or function, the image generation module 620 and the image generation module 320 may be modules of the same structure and/or function, and the LUT correction unit 342 in the correction module 630 and the correction module 340 may be modules of the same structure and/or function.

The obtaining module 610 may be configured to obtain at least one background event (for example, the at least one first background event) of the inherent radiating particle from the crystal(s) of the detector received by the detector of the imaging device. The image generation module 620 may be configured to determine the single-event image based on the at least one background event. The correction module 630 may be configured to correct the crystal position look-up table of the imaging device based on the single-event image. For more information about each module of the crystal position look-up table correction device 600, see FIG. 7 and its related description, which will not be repeated here.

FIG. 7 is an exemplary flowchart of a crystal position look-up table correction method according to some embodiments of the present application.

In some embodiments, the method 700 for correcting the crystal position look-up table of the imaging device may be performed by the system 100 for state detection of the imaging device (such as the processing device 140), the state correction device 300 of the imaging device, or the crystal position look-up table correction device 600. For example, the method 700 for correcting the crystal position look-up table may be stored in a storage device (such as the storage device 150) in a form of a program or instructions. When the system 100 for state detection (such as the processing device 140) executes the program or instructions, the method 700 for correcting the crystal position look-up table of the imaging device may be implemented. The operation diagram of the method 700 for correcting the crystal position look-up table presented below is illustrative. In some embodiments, the process may be accomplished with one or more additional operations not described and/or one or more operations not discussed. In addition, the order of operation of the method 700 for correcting the crystal position look-up table shown in FIG. 7 and described below is not intended to be restrictive. As shown in FIG. 7, the method 700 for correcting the crystal position look-up table may include the following operations.

In 710, a background event of a crystal of a detector of the imaging device may be obtained. In some embodiments, operation 710 may be performed by the processing device 140, the obtaining module 310, or the obtaining module 610.

In some embodiments, the background event may be related to the inherent radiating particle of the crystal. Specifically, if the crystal of the detector is the LYSO scintillation crystal, due to the existence of Lu176 in LYSO, a β particle generated in a decay may be immediately absorbed in the crystal of the detector where the decay occurs, and a γ photon may reach an opposite crystal through a whole field of vision and be absorbed by the corresponding detector crystal. After the crystal of the detector receives the β particle or the γ photon, the corresponding arrival time of the particle or the photon, a particle energy or photon energy, and the corresponding crystal position, i.e., background event information, may be recorded.

In some embodiments, before obtaining at least one background event of the inherent radiating particle(s) of the crystal(s) of the detector, the processing device 140 may set the energy window of the detector in advance, and obtain the at least one background event (for example, the at least one first background event) of the inherent radiating particle from the crystal(s) of the detector received by the detector based on a preset energy window. In some embodiments, the energy window of the detector may be determined based on an energy resolution. The higher the energy resolution, the smaller a value of a corresponding energy window. In some embodiments, to better adapt to the background inherent radiation phenomenon of the LYSO, received low-energy background radiation at least one particle may be filtered out by setting an appropriate energy window. In some embodiments, the value range of the energy window may be within the clinical threshold value range of the energy window of an imaging device (e.g., a PET device). As an example, we may first obtain a large amount of data on an energy window threshold value of a PET device in different hospitals in different regions during the actual application of the PET device, and the clinical threshold value range of the energy window of the PET device may be obtained through a large amount of data statistics. A range of an energy window of a preset detector may be within the threshold range of the clinical energy window of the corresponding PET device to ensure that data determined based on the obtained background events may match clinical data. In some embodiments, after determining the energy window of the imaging device, the processing device 140 may obtain a background event (for example, the first background event) based on the energy window.

In 720, a single-event image may be determined based on the background event. In some embodiments, operation 720 may be performed by the processing device 140, or the image generation module 320, or the image generation module 620.

In some embodiments, the processing device 140 may obtain a single-characteristic-energy-peak event based on the at least one background event; generate the single-event image based on the single-characteristic-energy-peak event. Specifically, the processing device 140 may form the corresponding energy spectrum based on particle energies or photon energies recorded after the detector in the at least one background event receives at least one β particle or at least one γ photon. In the process of attenuation, Lu176 may release at least one γ photon of three kinds of energy (i.e., 88 keV, 202 keV, and 307 keV) and at least one β particle with a random range of energy of 0 keV~597 keV, three kinds of at least one γ photon and at least one β particle may generate energy superposition between each other. Therefore, in some embodiments, a single characteristic peak event in the generated energy spectrum may include a characteristic energy peak event formed by an energy superposition of at least one photon of 88 keV and at least one β particle, a characteristic energy peak event formed by an energy superposition of at least one photon of 202 keV and at least one β particle, a characteristic energy peak event formed by at least one photon of 597 keV and/or by an energy superposition of at least one photon of 597 keV and at least one β particle, a characteristic energy peak event formed by an energy superposition of at least one photon of 307 keV and at least one β particle. Preferably, the single-characteristic-energy-peak event used in the embodiment may include the background event(s) of at least one photon of 597 keV received by the detector. In some embodiments, the processing device 140 may generate a single-event image based on the single characteristic peak event of at least one photon of 597 keV received by the detector of the imaging device 110.

Theoretically, the characteristic energy peak event of at least one photon of 597 keV may be configured to construct a single-event image. However, due to the scattering of at least one photon and the error caused by electronic measurement, actual measured data may have a certain broadened Gaussian distribution. Therefore, when constructing a single-event image, the processing device 140 may generate a single-event image based on the characteristic energy peak event of at least one photon of 597 keV and the characteristic energy peak event of at least one photon in a certain energy range around the characteristic energy peak event of at least one photon of 597 keV.

In 730, a crystal position look-up table of the imaging device may be corrected based on the single-event image. In some embodiments, operation 730 may be performed by the processing device 140, the correction module 340, or the correction module 630.

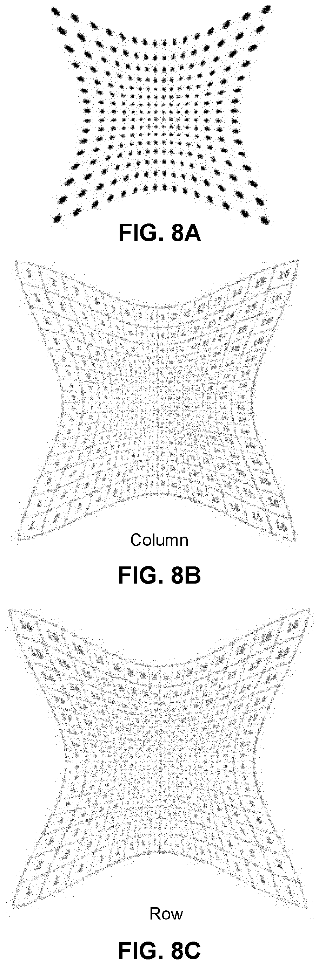

In some embodiments, the processing device 140 may obtain a pixel distribution corresponding to a crystal position label in the crystal position look-up table in the single-event image based on the single-event image; and/or correct the crystal position look-up table based on the pixel distribution corresponding to the crystal position label in the single-event image. The single-event image may represent an image formed by depositions of at least one photon of fixed energy received by a crystal array of the detector. For example, as shown in FIG. 8A, FIG. 8A is an exemplary single-event image. Each bright spot cluster in the single-event image shown in FIG. 8A represents mapping positions of crystals in the detector in the image. Each crystal of the detector may have a crystal position label based on the position of the crystal in the detector, that is, each bright spot cluster in the single-event image may have a crystal position label. Therefore, based on the single-event image, a corresponding relationship between each group of pixels (i.e., the bright spot cluster) in the single-event image and a crystal position label may be obtained. Based on the corresponding relationship between each group of pixels and the crystal position label in the single-event image, the crystal position look-up table of the imaging device may be corrected. For example, the processing device 140 may determine a corresponding number of columns shown in FIG. 8B and a corresponding number of rows are shown in FIG. 8C based on a count and/or positions of pixels of a bright spot cluster in the single-event image shown in FIG. 8A, and determine a corresponding crystal position label based on the number of rows and columns corresponding to each bright spot cluster in the single-event image, thus correcting the crystal position look-up table.

In some embodiments, the crystal(s) of the detector of the imaging device 110 may obtain the at least one background coincidence event generated by the background events based on the corrected crystal position look-up table, and the processing device 140 may generate a coincidence event image containing energy information of the background event based on the at least one background coincidence event. Based on the coincidence event image, the crystal position look-up table of the imaging device 110 may be secondary corrected to obtain the crystal position look-up table after a secondary correction. The crystal position look-up table after the secondary correction may more accurately locate a position of a crystal of the detector receiving the at least one background coincidence event.

In some embodiments, the processing device 140 may determine whether the crystal position look-up table of the imaging device is offset based on the at least one background event. In some embodiments, the processing device 140 may correct the crystal position look-up table based on the at least one background event in response to a deviation of the crystal position look-up table of the imaging device (that is, the state of the crystal position look-up table is abnormal). In some embodiments, the processing device 140 may obtain a single-event image and correct the crystal position look-up table of the imaging device based on the single-event image.

The above crystal position look-up table correction method may effectively avoid the use of radioactive sources in daily correction operations, simplify the correction process, effectively use background event data of the imaging device without patient scanning, improve a correction frequency, enable the imaging device to adaptively perform iterative correction optimization, and improve the performance of the imaging device.

Based on the method for correcting the crystal position look-up table provided by some embodiments of the present disclosure, background events with a low energy may be filtered by defining the energy window, the single event data of the detector may be collected, and the single-event image of the characteristic peak events of at least one photon of 597 keV generated by the natural decay of crystal Lu176 may be obtained. Compared the single-event image of the characteristic peak events of at least one photon of 597 keV with a single-event image of a characteristic peak event of at least one photon of 511 keV obtained from a radioactive source Na-22, the peak valley distribution of the two may be basically similar. Based on similar single-event images, the crystal position look-up table may be corrected, and the corrected crystal position look-up table may be used to collect the coincidence event of the detector of the imaging device, which may improve the imaging resolution of the imaging device.

In some embodiments, the corrected crystal position look-up table may be directly written into the imaging device, so that the crystal position look-up table may be directly used in subsequent scanning and imaging without further correction of the crystal position look-up table. In some embodiments, the corrected crystal position look-up table may be stored in the storage device (for example, the storage device 150), so that the crystal position may be corrected based on the crystal position look-up table during subsequent scanning imaging or image reconstruction.

An imaging device (such as the PET device) may drift in its energy gain state due to the aging of components and/or changes in the environment, which may affect the image quality in serious cases. This embodiment provides a method for detecting an energy correction state (or an energy state), which may use the background inherent radiation phenomenon of the crystal(s) of the detector to collect background events in a fixed energy interval under the condition of no radioactive source to detect the energy state.

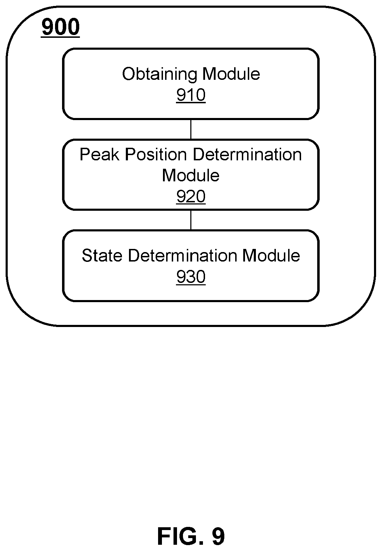

FIG. 9 is an exemplary block diagram of an energy state detection device according to some embodiments of the present application.

As shown in FIG. 9, the energy state detection device 900 may include an obtaining module 910, a peak position determination module 920, and a state determination module 930. In some embodiments, the obtaining module 910 and the obtaining module 310 may be modules of the same structure and/or function, the peak position determination module 920 and the peak position determination unit 332 may be modules of the same structure and/or function, and the state determination module 930 and the energy state correction unit 344 may be modules of the same structure and/or function. In some embodiments, the obtaining module 910 may be a module that has the functions of both the obtaining module 310 and the image generation module 320.

The obtaining module 910 may be configured to obtain at least one background event (e. g., the at least one first background event, or the at least one third background event) and related information of the crystal(s) of the detector of the imaging device 110. In some embodiments, the obtaining module 910 may be configured to form an energy spectrum based on the energy information of background events. In some embodiments, the obtaining module 910 may be configured to obtain the event-obtaining mode of the detector. For example, the obtaining module 910 may obtain an event-obtaining mode of the detector from an input device (e. g., an input device of the terminal device 130). In some embodiments, the obtaining module 910 may be configured to obtain the energy information of background events of the inherent radiating particle(s) of the crystal(s) of the detector received by the detector based on the event-obtaining mode and form an energy spectrum diagram. In some embodiments, the obtaining module 910 may send the obtained background event and its related information to other modules (for example, the image generation module 320), and other modules may generate the energy spectrum. The peak position determination module 920 may be configured to determine the peak position of the energy spectrum based on the energy spectrum. For example, the peak position determination module 920 may obtain at least two peak positions of the energy spectrum based on the energy spectrum. The state determination module 930 may be configured to determine the energy correction state based on the at least one peak position of the energy spectrum and the at least one corrected peak position corresponding to the at least one peak position of the energy spectrum. In some embodiments, the energy state detection device 900 may also include a correction module (for example, the correction module 340), which may be configured to correct the energy state of the imaging device after determining that the energy correction state of the imaging device is abnormal. For more information about each module of the energy state detection device 900, refer to FIG. 10 and its related description, which will not be repeated here.

FIG. 10 is an exemplary flowchart of an energy state detection method according to some embodiments of the present application.

In some embodiments, the energy state detection method 1000 of the imaging device may be executed by the system 100 for state detection of the imaging device (such as the processing device 140), the energy state correction unit 344, or the energy state detection device 900. For example, the energy state detection method 1000 may be stored in a storage device (such as the storage device 150) in the form of a program or instructions. When the system 100 for state detection (such as the processing device 140) executes the program or instructions, the energy state detection method 1000 of the imaging device may be implemented. The operation diagram of the energy state detection method 1000 of the imaging device shown below is illustrative. In some embodiments, the process may be accomplished with one or more additional operations not described and/or one or more operations not discussed. In addition, the sequence of operations of the energy state detection method 1000 shown in FIG. 10 and described below is not intended to be restrictive. As shown in FIG. 10, the energy state detection method 1000 may include the following operations.

In 1010, a background event of a crystal of a detector of the imaging device may be obtained. In some embodiments, operation 1010 may be performed by the processing device 140, the obtaining module 310, or the obtaining module 910.

In some embodiments, the processing device 140 may obtain at least one background event of the inherent radiating particle(s) of the crystal(s) of the detector received by the detector of the imaging device, such as the at least one first background event or the at least one third background event.

In some embodiments, the processing device 140 may determine the event-obtaining mode of the detector; obtain the at least one background event of the inherent radiating particle(s) of the crystal(s) of the detector received by the detector based on the event-obtaining mode. In some embodiments, the processing device 140 may determine the event-obtaining mode of the detector based on user input. Among them, the event-obtaining mode may include a single event mode and a coincident event mode.

In 1020, an energy spectrum may be generated based on the energy information of the background event. In some embodiments, operation 1020 may be performed by the processing device 140, the image generation module 320, or the obtaining module 910.

In some embodiments, the processing device 140 may generate the corresponding energy spectrum based on an energy of at least one particle or an energy of at least one photon recorded after the detector of the obtained imaging device receives at least one β particle or at least one γ photon. The energy spectrum may reflect a corresponding relationship between energies of at least one particle and a number of the background events. An abscissa of the energy spectrum is energy, and an ordinate of the energy spectrum is the number of background events corresponding to the energy.

In 1030, at least one peak position of the energy spectrum may be determined. In some embodiments, operation 1030 may be performed by the processing device 140, or the peak position determination unit 332, or the peak position determination module 920.

In some embodiments, the processing device 140 may obtain at least two peak positions of the energy spectrum based on the energy spectrum. Specifically, the peak position of the energy spectrum may include at least one of a peak positions of the full energy peak or a peak position of a single energy peak. The position of the full energy peak may be an energy peak position of at least one γ photon of 597 keV formed by superposition of three kinds of at least one γ photon. The position of a single energy peak may be an energy peak formed by the superposition of at least one β particle and at least one γ photon. For example: an energy peak position formed by the superposition of at least one photon of 88 keV and at least one β particle, an energy peak position formed by the superposition of at least one photon of 202 keV and at least one β particle, and/or an energy peak position formed by the superposition of at least one photon of 307 keV and at least one β particle.

More specifically, if the energy spectrum is formed by energy information of the background single event of the single event mode, the position of the full energy peak and the position of the single energy peak may be obtained. At least two peak positions of the energy spectrum may be at least two of: an energy peak position of 597 keV, an energy peak position formed by the superposition of at least one photon of 88 keV and at least one β particle, an energy peak position formed by the superposition of at least one photon of 202 keV and at least one β particle, and an energy peak position formed by the superposition of at least one photon of 307 keV and at least one β particle. If the energy spectrum is formed by the energy information of the at least one background coincidence event with the event mode, a position of the single energy peak may be obtained. At least two peak positions of the energy spectrum may be at least two of: an energy peak position formed by the superposition of at least one photon of 88 keV and at least one β particle, an energy peak position formed by the superposition of at least one photon of 202 keV and at least one β particle, and an energy peak position formed by the superposition of at least one photon of 307 keV and at least one β particle.

In 1040, an energy correction state may be determined based on the at least one peak position of the energy spectrum and at least one corrected peak position corresponding to the at least one peak position of the energy spectrum. In some embodiments, operation 1040 may be performed by the processing device 140, or the energy state correction unit 344, or the state determination module 930.

Specifically, in some embodiments, the processing device 140 may first obtain at least one corrected peak position corresponding to at least one peak position of a current energy spectrum before determining the energy correction state of the imaging device. The at least one corrected peak position may correspond to a peak position of the energy spectrum formed by at least one photon of 511 keV. The at least one corrected peak position may refer to a peak position of the energy spectrum formed by at least one γ photon of 511 keV energy emitted by a radiation source (such as FDG (fluorodeoxyglucose) and Ge68 (germanium-68)) in a traditional method for correcting an imaging device (e.g., the PET device).

In some embodiments, the processing device 140 may calculate a ratio of the at least one peak position of the energy spectrum to the at least one corrected peak position of the energy spectrum; and compare the ratio with a preset threshold value. If the ratio is different from the preset threshold value, it may be determined that the energy correction state of the imaging device is abnormal. Under the condition that the energy of the imaging device is unchanged, the ratio of the peak position of the energy spectrum to the at least one corrected peak position of the energy spectrum may be a stable relationship. The relationship may be expressed as a fixed value, or as a look-up table where the energy peak position is related to the ratio. When the energy of the imaging device changes, the energy peak position of the energy spectrum of the at least one background event may change. Further, a ratio relationship between the peak position of the energy spectrum and the at least one corrected peak position may change. Therefore, the energy state, that is, the energy drift, may be judged by the ratio relationship between the peak position of the energy spectrum and the at least one corrected peak position. More specifically, a large amount of data may be counted in advance to obtain the ratio relationship between the peak position and the at least one corrected peak position of the energy spectrum of the current imaging device. Based on a large amount of ratio relationship data, a ratio threshold value between different peak positions of the corresponding imaging device and the at least one corrected peak positions may be obtained. In practical application, the peak position of the energy spectrum may be compared with the at least one corrected peak position to obtain the ratio; then a ratio threshold value of the peak position of the energy spectrum corresponding to the ratio may be obtained. A calculated actual ratio may be compared with the ratio threshold value. If the ratio is different from the corresponding ratio threshold value, it may be judged that the energy state is abnormal, that is, the current imaging device may have energy drift; If the ratio is the same as the corresponding ratio threshold value, it may be judged that the energy state is normal, that is, the current imaging device energy may not drift.

Based on the above method, the background events may be collected through passive detection and the energy state of the imaging device may be detected, which may reduce the radiation dose received by the operator and reduce the use cost of the hospital. Compared with active detection, the above method may be simple to operate and may be detected at any time.

In some embodiments, the processing device 140 may adjust the system parameters of the imaging device in response to an abnormal energy correction state of the imaging device to correct the energy state of the imaging device. In some embodiments, the processing device 140 may send prompt information to the user in response to the abnormal energy correction state of the imaging device.

In some embodiments, the event-obtaining mode may be a single event mode, and the processing device 140 may obtain the energy information of the background single event received by the detector and form an energy spectrum. The energy spectrum may include at least one of a peak value of a full energy peak or a peak value of a single energy peak.

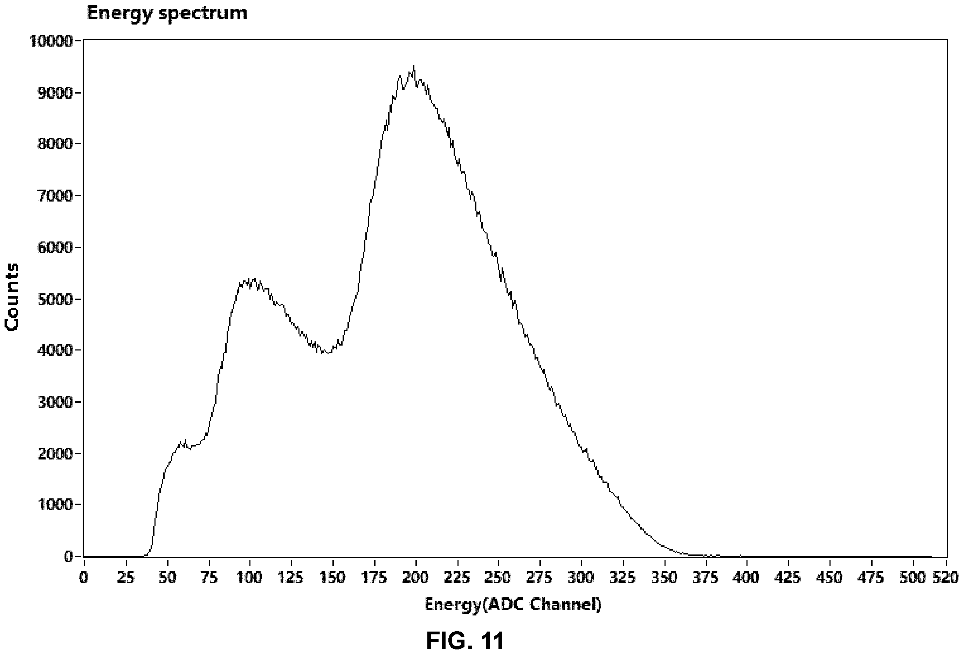

Specifically, in the single event mode, the energy spectrum generated by energy information of the background single event received by the detector may include the energy peak position of 597 keV, the energy peak position formed by the superposition of at least one photon of 88 keV and at least one β particle, the energy peak position formed by the superposition of at least one photon of 202 keV and at least one β particle, and/or the energy peak position formed by the superposition of at least one photon 307 keV and at least one β particle. More specifically, in the single event mode, as shown in FIG. 11, FIG. 11 is an exemplary energy spectrum generated in the single event mode. The peak position is the peak position of the full energy peak with an energy of 597 keV. For the superposition effect of at least one β particle, the actual energy value at the position may be greater than 597 keV, about 635 keV. By changing the ratio of the peak position of the full energy peak to the at least one corrected peak position, it may be possible to determine the energy correction state of the imaging device (e.g., a PET device). In this embodiment, the peak position with an energy of 597 keV is taken as an example. In some alternative embodiments, the energy peak position formed by the superposition of at least one photon of 88 keV and at least one β particle, the energy peak position formed by the superposition of at least one photon of 202 keV and at least one β particle, and/or the energy peak position formed by the superposition of at least one photon of 307 keV and at least one β particle may be obtained to determine the energy correction state, which is not limited to the present disclosure.

In some embodiments, the event-obtaining mode may be a coincidence event mode. The processing device 140 may obtain information received by the crystal(s) of each detector based on a preset time window. The information may include energy information of at least one particle and/or at least one photon and the arrival time of at least one particle and/or at least one photon. The processing device 140 may filter the photon energy information based on the arrival time of the at least one photon. The energy spectrum may be generated based on the filtered photon energy information, and the energy spectrum may include a peak value of a single energy peak.

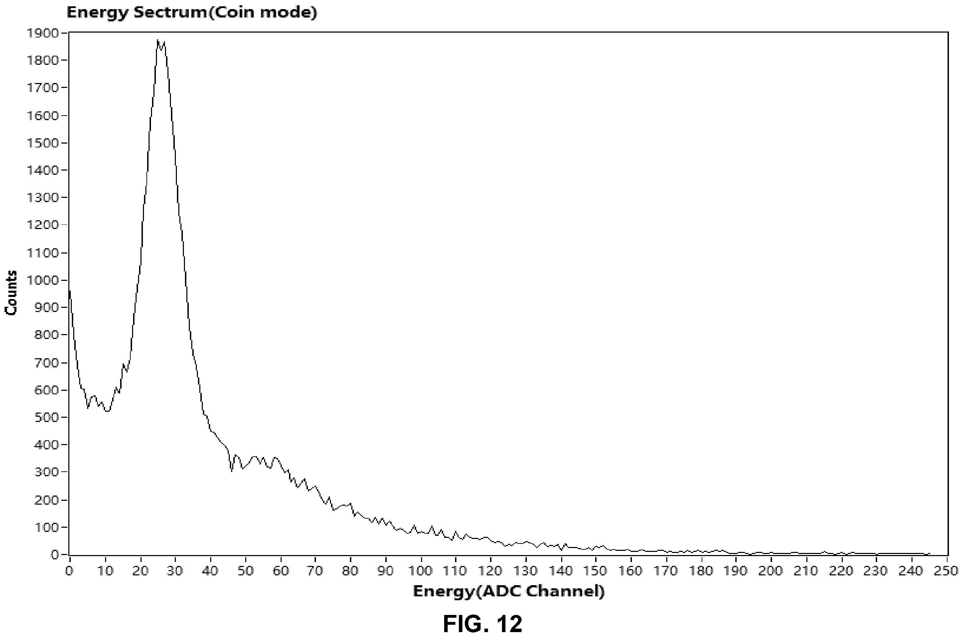

The time window may be configured to reflect the sensitivity of an imaging device (for example, a PET device). Preferably, the range of the time window may not be less than the range of the clinical time window threshold value of the imaging device. In some embodiments, the processing device 140 may determine the range of the time window based on the time resolution. For example, the value of the time window may be greater than the value of the time resolution, and the value of the time window may be determined by 1/10 of a height and width of a time distribution curve. The time resolution may refer to a half height and full width of a time difference distribution corresponding to two detectors in the known at least one background coincidence event. In order to better adapt to the inherent radiation phenomenon of the LYSO background, an appropriate time window may be set. The at least one particle received by the crystal(s) of each detector may include at least one β particle, and/or at least one γ photon. The at least one β particle may be absorbed after generation, and the absorption time may be expressed as Ta, while at least one γ photon may reach the crystal of the opposite detector through the whole field of vision and be detected and absorbed, the absorption time may be expressed as Tb, and thus a detection time of the γ event may be longer than a detection time of the β event, that is, Ta<Tb. Based on this principle, the γ event of the crystal may be filtered. The photon energy information may be filtered based on the arrival time of at least one photon, that is the γ event may be filtered. Then, the energy spectrum may be formed based on the filtered photon energy information. FIG. 12 shows the energy spectrum generated based on the coincidence event mode in one embodiment. As shown in FIG. 12, the filtered peak value of the energy spectrum of at least one γ photon is relatively clear, and the corresponding energy of the peak position is 307 keV. By analyzing the change of the ratio of the peak position with the energy of 307 keV to the peak position with the correction energy of 511 keV, the current energy state of the imaging device may be obtained. In some embodiments, it is also possible to filter at least one peak position of the energy spectrum formed by at least one γ photon with three kinds of energy of 88 keV, 202 keV, and/or 307 keV.

Based on the above method of generating the energy spectrograms, energy spectrograms under different event-obtaining modes may be obtained based on different event-obtaining modes, and more accurate energy peak positions may be further obtained. Based on the single event mode for obtaining, the position of the full energy peak with an energy of 597 keV in the crystal energy spectrum of a single event may be directly detected, but the invention is not limited to the full energy peak. Based on the event mode, the peak position with an energy of 307 keV or a peak position with any other energy may be detected, making the subsequent determination of the energy state more accurate.

The energy scale may refer to a conversion operation of mapping the ADC value referring to the particle energy collected by the imaging device (such as a PET device) to an actual energy. After receiving at least one photon, the detector of the imaging device may excite at least one visible light photon, which may be converted into a corresponding electrical signal through a photomultiplier tube (PMT) or a silicon photomultiplier (SiPM), and the electrical signal may be converted into ADC values through an analog-digital conversion. By mapping a known energy with the corresponding ADC value, the energy scale may be completed and the energy scale curve may be obtained. Among them, the ADC value may be a value obtained after the analog-digital conversion of the collected electrical signal.

The method for determining the energy scale curve provided in this embodiment may obtain the background event(s) of at least one γ photon through the inherent radiation phenomenon of the crystal(s) of the detector, at least two characteristic energy peaks of characteristic energy peaks such as a characteristic energy peak with an energy of 307 keV and a characteristic energy peak with an energy of 597 keV, etc., may be obtained in an energy spectrum of the at least one background event obtained by the crystal. The characteristic energy peak with an energy of 511 keV in a practical application may be performed an interpolation and a fitting by the at least two characteristic energy peaks to obtain a corresponding relationship between an ADC value and an actual photon energy.

FIG. 13 is an exemplary block diagram of a device for determining an energy scale curve according to some embodiments of the present disclosure.

As shown in FIG. 13, the energy scale curve determination device 1300 may include an obtaining module 1310, an event filtering module 1320, and a curve determination module 1330. In some embodiments, the obtaining module 1310 and the obtaining module 310 may be modules with the same structure and/or function, and the energy state correction unit 344 may have functions of both the event filtering module 1320 and the curve determination module 1330.

The obtaining module 1310 may be configured to obtain the background events (for example, the third background event) of the inherent radiating particle of the crystal received by the detector. The event filtering module 1320 may be configured to filter the background events and determine at least two energy peaks related to the nuclide decay of the crystal and ADC values corresponding to the at least two energy peaks. The curve determination module 1330 may be configured to determine the energy scale curve based on the at least two energy peaks and the ADC values corresponding to the at least two energy peaks. In some embodiments, the energy scale curve determination device 1300 may also include an extraction module. The extraction module may be configured to determine the ADC value corresponding to any energy peak on the energy scale curve based on the energy scale curve. In some embodiments, the extraction module may also be configured to determine the ADC value corresponding to the energy peak value with an energy of 511 keV based on the energy scale curve. For more information about each module of the energy scale curve determination device 1300, see FIG. 14 and its related description, which will not be repeated here.

FIG. 14 is an exemplary flowchart of a process for determining an energy scale curve according to some embodiments of the present disclosure.

In some embodiments, the method 1400 for determining the energy scale curve of the imaging device may be performed by the system 100 for state detection of the imaging device (such as the processing device 140), the energy state correction unit 344, or the energy scale curve determination device 1300. For example, the method 1400 for determining the energy scale curve may be stored in a storage device (such as the storage device 150) in the form of a program or instruction. When the system 100 for state detection (such as the processing device 140) executes the program or instruction, the method 1400 for determining the energy scale curve may be implemented. The operation diagram of the method 1400 for determining the energy scale curve presented below is illustrative. In some embodiments, the process may be accomplished with one or more additional operations not described and/or one or more operations not discussed. In addition, an order of an operation of the method 1400 for determining the energy scale curve shown in FIG. 14 and described below is not intended to be restrictive. As shown in FIG. 14, the method 1400 for determining the energy scale curve may include following operations.

In 1410, a background event of a crystal of a detector of the imaging device may be obtained. In some embodiments, operation 1410 may be performed by the processing device 140, or the obtaining module 310, or the obtaining module 1310.

In some embodiments, the processing device 140 may obtain at least one background events (for example, the at least one first background event or the at least one third background event) received by the detector related to the inherent radiating particle(s) of the crystal(s) of the detector. The imaging device may collect the at least one background event in a passive state during idle time. Due to a high proportion of various scattering events in the background, it may be easy to reduce a signal-to-noise ratio of a γ characteristic energy peak in an energy spectrum of the at least one background event. Therefore, the longer the theoretical obtaining time for background events, the better. The obtaining time of the at least one background event may be within a range of 0.1-20 hours. For example, the obtaining time may be no less than 30 minutes.

In some embodiments, the time window and the energy window of the imaging device may be set in advance; the background events may be acquired based on the time window and the energy window. For more information about the energy window, please refer to the relevant description in FIG. 7, and for more information about the time window, please refer to the relevant description in FIG. 10, which will not be repeated here.

In 1420, at least two energy peak values related to a nuclide decay of the crystal, and ADC values corresponding to the at least two energy peak values may be determined based on the background event. In some embodiments, operation 1420 may be performed by the processing device 140, the event filtering module 1320, or the energy state correction unit 344.

In some embodiments, the processing device may determine a characteristic energy peak value of γ decay of the nuclide of the crystal based on the at least one background event; perform an analog-to-digital conversion on the characteristic energy peak value of γ decay of the nuclide to obtain the ADC value corresponding to the characteristic energy peak value; select at least two energy peak values in the characteristic energy peak values of γ decay and the ADC values corresponding to the at least two energy peak values. Specifically, the processing device 140 may generate a corresponding energy spectrogram based on the at least one background event, and obtain the characteristic energy peak value of γ decay of the nuclide by performing energy filtering on the energy spectrogram. In some embodiments, the nuclide may include a radioactive nuclide present in the crystal(s) of a detector such as Lu176. Taking the nuclide as Lu176 as an example, the characteristic energy peaks of γ decay corresponding to Lu176 may include: a characteristic energy peak of the 88 keV at least one photon of γ decay, a characteristic energy peak of the 202 keV at least one photon of γ decay, a characteristic energy peak of the 307 keV at least one photon of γ decay, a characteristic energy peak of the 597 keV photon of γ decay, and characteristic energy peaks corresponding to various energy values of γ decay. By performing the analog-to-digital conversion on the obtained peak value of characteristic energy peak of γ decay, the ADC value of the corresponding peak value of characteristic energy peak of γ decay may be obtained. After obtaining all the characteristic energy peaks of γ decay and the ADC values of the corresponding characteristic energy peaks of γ decay, some characteristic energy peaks of γ decay and ADC values corresponding to the characteristic energy peaks may be selected therefrom. In a process of scaling energy scales, the more characteristic energy peaks of γ decay and ADC values corresponding to the characteristic energy peaks be scaled, the more accurate obtained energy scales. In some embodiments, preferably, at least two characteristic energy peaks (e.g., 0 keV, 307 keV, 597 keV, etc.) and ADC values corresponding to the corresponding characteristic energy peaks of γ decay may be selected.

In 1430, the energy scale curve may be determined based on the at least two energy peak values and the ADC values corresponding to the at least two energy peak values. In some embodiments, operation 1430 may be performed by the processing device 140, or the curve determination module 1330, or the energy state correction unit 344.

In some embodiments, the processing device 140 may determine the energy scale curve through interpolation fitting. Specifically, the processing device 140 may perform interpolation fitting on a plurality of ADC values and the corresponding characteristic energy peaks of γ decay to obtain an energy scale curve. For example, the processing device 140 may perform interpolation fitting based on the selected three characteristic energy peaks of γ decay of 0 keV, 307 keV and 597 keV, and the ADC values corresponding to the characteristic energy peaks of γ decay to obtain an energy scale curve. The energy scale curve may cover all energy ranges, for example, an energy range of the energy scale curve may be from 0 to 2000 keV.

In some embodiments, after the energy scale curve is obtained based on the plurality of ADC values and the characteristic energy peaks of γ decay of the corresponding nuclides, an ADC value corresponding to any energy peak value on the energy scale curve may be determined based on the energy scale curve. That is, all energy peak values and corresponding ADC values may be obtained based on the energy scale curve. In an actual use process, various drugs (with various characteristic peak nuclides) may be used in the actual use of the imaging device. Accordingly, at least one photon of corresponding energy may be detected. Therefore, it is necessary to obtain all the characteristic energy peak values and corresponding ADC values in advance. For example, after obtaining the energy scale curve based on the plurality of ADC values and the characteristic energy peaks of γ decay of the corresponding nuclides, the ADC value corresponding to the energy peak value of 511 keV may be determined based on the energy scale curve. In some embodiments, the energy peak of 511 keV may be a characteristic peak generated by a commonly used radionuclide injection. Therefore, at least one ADC value corresponding to the at least one energy peak of 511 keV may be obtained in advance based on the energy scale curve.

The above method for determining an energy scale curve may avoid the use of radioactive sources, improve the safety and feasibility of operation, effectively use background event data of a PET imaging device without patient scanning, and more effectively and timely update the energy scale curve of the detector.

In some embodiments, a method for correcting at least one energy of the crystal(s) of the detector of the imaging device is also provided. Based on the energy scale curve, the ADC value corresponding to the energy peak value of 511 keV may be determined, so that at least one photon energy received by the detector may be corrected, so that the detector may judge the received energy more accurately, and further improve the imaging accuracy of the imaging device.

When the detector of the imaging device (such as the PET device) receives a pair of γ photons generated in positron annihilation, a corresponding detector module may need to measure a time when the pair γ photons arrive at the crystal(s) of the detector. In order to ensure the consistency of the time, the time of each detector module may need to be aligned. In addition, a clock may be out of synchronization after a time alignment. Therefore, it may be necessary to perform the time-synchronization on each detector module.

In traditional technology, cables may be configured to perform the time-synchronization, each detector module may be connected with the cables, and each detector module may be sent pulses through the cables at the same time. The detector module may set a local clock to zero based on the pulse to complete a clock synchronization. The above traditional technologies may not detect a clock synchronization state, and when the cable is aging or the interface is loose, it may cause a problem of clock synchronization failure.

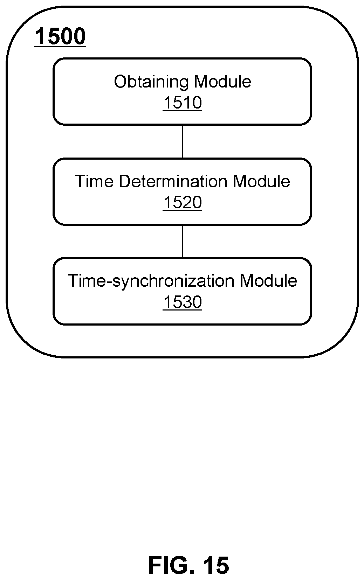

The present disclosure provides a method for time synchronization, which may use the background inherent radiation of the crystal(s) of the detector to collect the corresponding background events under a condition of no radioactive source, determine the measured time of flight and theoretical time of flight based on the information of the background events, and perform the time-synchronization on at least one detector module of the detector based on the measured time of flight and the theoretical time of flight. FIG. 15

FIG. 15 is an exemplary block diagram of a time-synchronization device according to some embodiments of the present disclosure.

As shown in FIG. 15, the time-synchronization device1500 may include an obtaining module 1510, a time determination module 1520, and a time-synchronization module 1530. In some embodiments, the obtaining module 1510 and the obtaining module 310 may be modules with the same structure and/or function, the time determination module 1520 and the time determination unit 334 may be modules with the same structure and/or function, and the time-synchronization module 1530 and the time-synchronization unit 346 may be modules with the same structure and/or function.

The obtaining module 1510 may be configured to obtain the at least one background coincidence event and related information of the crystal(s) of the detector of the imaging device, and the at least one background coincidence event may be related to the inherent radiating particle of the crystal. The time determination module 1520 may be configured to calculate the measured time of flight and the theoretical time of flight based on the background coincidence event information. The time-synchronization module 1530 may be configured for performing a time-synchronization on the detector based on the measured time of flight and the theoretical time of flight. For more information about each module of the time-synchronization device1500, refer to FIG. 16 and its related description, which will not be repeated here.

FIG. 16 is an exemplary flowchart of a time-synchronization method according to some embodiments of the present disclosure.

In some embodiments, the time-synchronization method 1600 of the imaging device may be executed by the system 100 for state detection of the imaging device (such as the processing device 140) or the time-synchronization device1500. For example, the time-synchronization method 1600 may be stored in a storage device (such as a storage device 150) in a form of a program or an instruction, and may be implemented when the system 100 for state detection (such as the processing device 140) executes the program or instruction. The operation diagram of the time-synchronization method 1600 presented below is illustrative. In some embodiments, the process may be accomplished with one or more additional operations not described and/or one or more operations not discussed. In addition, the sequence of operations of the time-synchronization method 1600 shown in FIG. 16 and described below is not intended to be restrictive. As shown in FIG. 16, the time-synchronization method 1600 may include the following operations.

In 1610, at least one background coincidence event of the crystal of the detector of the imaging device may be obtained. In some embodiments, operation 1610 may be performed by the processing device 140, or the obtaining module 310, or the obtaining module 1510.

In some embodiments, the processing device 140 may obtain information about the at least one background coincidence event (for example, the at least one background coincidence event based on coincidence processing of the at least one first background event and the at least one second background event, or the at least one third background event, or the at least one third background event) related to the inherent radiating particle of the crystal of the detector received by the detector of the imaging device.

In 1620, the measured time of flight and the theoretical time of flight may be determined based on the background coincidence event information. In some embodiments, operation 1620 may be performed by the processing device 140, the time determination unit 334, or the time determination module 1520.

In some embodiments, the processing device 140 may obtain the measured time of flight of the corresponding background coincidence event based on a time when two particles in each background coincidence event are detected by the crystal in the detector module (that is, an arrival time reaching the detector); obtain the theoretical time of flight of the corresponding background coincidence event based on crystal positions of two particles in each background coincidence event. FIG. 17 is a schematic diagram of a detector module receiving at least one photon according to some embodiments of the present disclosure. As an example only, as shown in FIG. 17, if a time of a crystal in one of the detector modules of the detector receives a first particle (e.g., at least one β particle) is T1, and a time of a crystal in another detector module receives a second particle (for example, γ photon) is T2, the measured time of flight of the corresponding event may be obtained through subtracting the time T1 of receiving the first particle from the time T2 of receiving the second particle. If a crystal of the detector receiving the first particle is A and a crystal of the detector receiving the second particle is B, then a linear distance L between the crystal A of the detector and the crystal B of the detector may be obtained, and then the linear distance L may be divided by a speed of light C to obtain the theoretical time of flight.

In 1630, a time-synchronization may be performed on the detector based on the measured time of flight and the theoretical time of flight. In some embodiments, operation 1630 may be performed by the processing device 140, the time-synchronization unit 346, or the time-synchronization module 1530.

In some embodiments, the processing device 140 may determine that times between each detector module of the imaging device are not synchronized in response to a difference between the measured time of flight and the theoretical time of flight of the corresponding background coincidence event (for example, a difference between the measured time of flight and the theoretical time of flight may be greater than a threshold value); determine that the times between each detector module of the imaging device are synchronized in response to a fact that the measured time of flight is the same as the theoretical time of flight of the corresponding background coincidence event (for example, the difference between the measured time of flight and the theoretical time of flight may be not greater than a threshold value). In some embodiments, the threshold value may be a clock cycle of the detector module. In some embodiments, if the times between each detector module of the imaging device are not synchronized, the processing device 140 may perform the time-synchronizations on each detector module of the detector of the imaging device based on the measured time of flight and the corresponding theoretical time of flight.

In some embodiments, the processing device 140 may determine the time difference based on the measured time of flight and the theoretical time of flight; perform a time synchronization for each detector module of the imaging device based on the time difference. For example, the processing device 140 may obtain the time difference by subtracting the theoretical time of flight from the measured time of flight (or obtain the time difference by subtracting the measured time of flight from the theoretical time of flight), and perform the time synchronization on each detector module of the imaging device based on an absolute value of the time difference.

In some embodiments, the processing device 140 may calculate a plurality of differences between the plurality of measured values of time of flight and the plurality of theoretical values of time of flight based on the plurality of measured values of time of flight and the plurality of theoretical values of time of flight received by any pair of detector modules of the detector of the imaging device; based on the plurality of differences, calculate a mean value of the differences, and determine the mean value as the time difference. Specifically, in order to ensure the reliability of data, the plurality of differences between the plurality of measured values of time of flight and the plurality of theoretical values of time of flight may be calculated for the same pair of detector modules, that is, information of the plurality of background consistent events detected by the same pair of detector modules may be obtained first. For a time when two particles in the information of each background coincidence event are detected by the crystal in the detector module (that is, the time when the two particles arrive at the detector module) and a crystal position in the detector module where the plurality of background coincidence events is detected, the measured time of flight and the theoretical time of flight may be calculated, and then a difference between the measured time of flight and the theoretical time of flight of the corresponding background coincidence event may be calculated; the time difference may be determined by averaging differences of the plurality of background coincidence events, and then the crystals of each detector module of the imaging device may be performed the time-synchronization through the time difference. This method may make time synchronization more accurate.

In some embodiments, the time difference may be greater than or equal to one clock cycle. Each detector module of the detector of the imaging device may include a counter, which may be configured as the clock of all detector crystals in the corresponding detector module to record the current time. That is, the crystal(s) of the detectors belonging to the same detector module may use the same clock. The counter may be incremented by 1 for each cycle. A process of a clock synchronization may be a process of counter-alignment. Synchronize the clock of each crystal of the detector, that is, synchronize the clock of the corresponding detector module (for example, align the counters of each detector module). Specifically, if the time difference is greater than or equal to one clock cycle, the processing device 140 may adjust the value of the counter of one detector module in the pair of detector modules, and determine the adjusted value of the counter as a time reference standard. In some embodiments, if the time difference is less than one clock cycle, it may be not necessary to adjust the corresponding counter; if the time difference is greater than or equal to one clock cycle, a counter of any detector module in counters of a pair of detector modules may be adjusted, for example, the counter may be added 1 or subtracted 1 so that the counter may be the same as a counter of another detector module in the pair of detector modules. After the adjustment is completed, the background coincidence event information of the inherent radiating particle(s) of the crystal(s) of the detector may be obtained, and then a time difference based on the background coincidence event information may be calculated. If the time difference is less than one clock cycle, the clock synchronization may be completed; If the time difference is greater than or equal to one clock cycle, the counter may be continued to adjust until a detected time difference is less than one clock cycle.

In some embodiments, one of the detector modules in any pair of detector modules may be used as a reference module to perform the time-synchronization on other detector modules in the imaging device except for the pair of detector modules until a time difference between all detector modules is less than one clock cycle. Specifically, in the process of the clock synchronization, the clocks of any pair of detector modules in the detector may be synchronized first. After the clock synchronization, any detector module in synchronized pair of detector modules may be used as the reference module, and then all remaining detector modules may be synchronized in turn until the time difference between all detector modules is less than one clock cycle.

In some embodiments, the imaging device may include a control module, which may output a control signal based on a comparison result between the time difference and a clock cycle. If the time difference is greater than or equal to a clock cycle, the control module may generate an adjustment signal to adjust the counters of each detector module to achieve clock alignment; if the time difference is less than one clock cycle, no adjustment signal may be generated. In this method, the background inherent radiation of the crystal(s) of the detector may be used to synchronize the clocks between detector modules, and a synchronization state may be monitored in real-time, which may simplify the structure of the imaging device, increase the reliability of the device, and avoid a clock synchronization failure caused by the cables and other reasons.

Due to inherent characteristics of the hardware, the state of time of flight of the imaging device (such as the PET device) may drift with time, which may affect the imaging quality in serious cases. The embodiment of the present disclosure provides a method for detecting a state of time of flight. Under a condition of no radiation source, the at least one background coincidence event may be collected by using the background inherent radiation phenomenon of the crystal(s) of the detector, and the state of time of flight of the imaging device may be corrected based on the background coincidence event information, which may solve problems of a high radiation dose to the operator, a high hospital cost, and a complex operation, etc.

FIG. 18 is an exemplary block diagram of a time-of-flight state detection device according to some embodiments of the present application.

As shown in FIG. 18, the time-of-flight state detection device 1800 may include an obtaining module 1810, a time of flight determination module 1820, and a state determination module 1830. In some embodiments, the obtaining module 1810 and the obtaining module 310 may be modules with the same structure and/or function, the time of flight determination module 1820 and the time determination unit 334 may be modules with the same structure and/or function, and the state determination module 1830 and the TOF state correction unit 348 may be modules with the same structure and/or function.

The obtaining module 1810 may be configured to obtain the at least one background coincidence event (for example, the third background event or the fourth background event) and related information. In some embodiments, the obtaining module 1810 may also be configured to determine the time window and energy window of the imaging device; obtain at least one background coincidence event based on the time window and the energy window. The time of flight determination module 1820 may be configured to calculate the measured time of flight and the theoretical time of flight based on the background coincidence event information. The state determination module 1830 may be configured to determine the state of time of flight of the imaging device based on the measured time of flight and the theoretical time of flight.

In some embodiments, the time-of-flight state detection device 1800 may also include a correction module. The correction module may be configured for correcting to obtain a corrected time of flight based on the flight time of the imaging device. In some embodiments, the correction module may also be configured to obtain an energy-time mapping relationship; the energy-time mapping relationship may reflect a mapping relationship between a particle energy and a time-of-flight offset; based on an energy of two particles in the at least one background coincidence event and the energy-time mapping relationship, obtain the time-of-flight offset of the corresponding background coincidence event; the time of flight of the imaging device may be corrected based on the of the time-of-flight offset to obtain the corrected time of flight.

FIG. 19 is an exemplary flowchart of a method for detecting a state of time of flight according to some embodiments of the present disclosure.

In some embodiments, the state of time of flight (i.e., TOF state) detection method 1900 of the imaging device may be performed by the system 100 for state detection of the imaging device (such as the processing device 140) or the time-of-flight state detection device 1800. For example, the time-of-flight state detection method 1900 may be stored in a storage device (such as the storage device 150) in a form of a program or instruction. When the system 100 for state detection (such as the processing device 140) executes the program or instruction, the time-of-flight state detection method 1900 may be implemented. The operation diagram of the time-of-flight state detection method 1900 shown below is illustrative. In some embodiments, the process may be accomplished with one or more additional operations not described and/or one or more operations not discussed. In addition, a sequence of operations of the time-of-flight state detection method 1900 is shown in FIG. 19 and described below is not intended to be restrictive. As shown in FIG. 19, the time-of-flight state detection method 1900 may include the following operations.

In 1910, a background coincidence event of the crystal of the detector of the imaging device may be obtained. In some embodiments, operation 1910 may be performed by the processing device 140, the obtaining module 310, or the obtaining module 1810.

In some embodiments, the processing device 140 may obtain the background coincidence event (for example, the third background event or the fourth background event) related to the inherent radiating particle of the crystal of the detector received by the detector of the imaging device. In some embodiments, the processing device 140 may determine the time window and energy window of the imaging device; Based on the time window and energy window, the background coincidence event and related information of the inherent radiating particle of the crystal received by the detector may be obtained. For more information about each module of the energy scale curve determination device 1300, see FIG. 14 and its related description, which will not be repeated here. In some embodiments, the processing device 140 may obtain the background coincidence event through coincidence processing based on two different sets of background single events (for example, the at least one first background event and the at least one second background event).

In 1920, the measured time of flight and the theoretical time of flight may be determined based on the background coincidence event information. In some embodiments, operation 1920 may be performed by the processing device 140, or the time determination unit 334, or the time of flight determination module 1820.

In some embodiments, the processing device 140 may obtain the measured time of flight of the corresponding background coincidence event based on the arrival time of two particles in the background coincidence event; obtain the theoretical time of flight of the corresponding background coincidence event based on crystal positions of the two particles in the background coincidence event. For more information about the measured time of flight and theoretical time of flight, please refer to FIG. 16 and its related description, which will not be repeated here.

In 1930, the state of the time of flight of the imaging device based on the measured time of flight and the theoretical time of flight may be determined, and the state of the time of flight may reflect whether the crystal of the detector has drifted. In some embodiments, operation 1930 may be performed by the processing device 140, or a time-of-flight state correction unit, or the state determination module 1830.

In some embodiments, the processing device 140 may compare the difference between the measured time of flight and the theoretical time of flight. In response to the difference between the measured time of flight and the theoretical time of flight exceeding a threshold, the processing device 140 may determine that a crystal corresponding to the background coincidence event has drifted, that is, the state of time of flight is abnormal. Specifically, if the measured time of flight is different from the theoretical time of flight of the corresponding background coincidence event (for example, the difference between the measured time of flight and the theoretical time of flight is greater than a threshold), it may be determined that the crystal(s) of the detector corresponding to the background coincidence event has drifted; If the measured time of flight is the same as the theoretical time of flight of the corresponding background coincidence event (for example, the difference between the measured time of flight and the theoretical time of flight is not greater than a threshold), it may be considered that the crystal(s) of the detector corresponding to the background coincidence event is normal without drift. Theoretically, when a TOF correction state (i.e., TOF state) of the imaging device is normal, the measured time of flight and the theoretical time of flight may be equal, that is, a difference between the measured time of flight and the theoretical time of flight is close to zero. In some embodiments, the processing device 140 may confirm whether the corresponding crystals are offset by calculating the difference between the measured time of flight and the theoretical time of flight of all background coincidence events on the crystals of each detector, based on whether the average value of the differences of all the background coincidence event or an expected value after Gaussian fitting exceeds a threshold.

In some embodiments, the processing device 140 may correct the flight time of the detector based on the background coincidence event information after determining that the state of time of flight is abnormal, and obtain the corrected time of flight.

Specifically, the processing device 140 may obtain an energy-time mapping relationship. The energy-time mapping relationship may reflect a corresponding relationship between a particle energy and a time-of-flight offset. The time-of-flight offset of the corresponding background coincidence event may be obtained based on the energy of two particles of each background coincidence event and the energy-time mapping relationship. The time of flight of the detector may be corrected based on the time-of-flight offset to obtain the corrected flight time. In some embodiments, the background coincidence event information may include the energy of two particles. In combination with FIG. 5, crystal array A and crystal array B are respectively coupled with optical sensors, and an output signal is amplified by an amplifier to obtain time information Ta of event β and time information Tb of event γ, and energy information Ea and Eb of the two events may be obtained through an integration, that is, the energy of the two particles. Before the correction, the energy-time mapping relationship may be established in advance. Specifically, based on the imaging device (e.g., a PET device) that has completed the TOF correction, it may be possible to count the corresponding time-of-flight offset of a large number of particles with different energies whose background matches the event, and establish the corresponding relationship between the energy of two particles and the time-of-flight offset, that is, the energy-time mapping relationship. In an actual use, a pre-established energy-time mapping relationship may be first obtained, and then the energy of the two particles in the background coincidence event may be obtained. The energy-time mapping relationship may be obtained by the energy of the two particles in the background coincidence event, the time-of-flight offset corresponding to the energy of the two particles may be determined, the time of flight of the detector may be corrected based on the time-of-flight offset, and the corrected time of flight may be obtained.

The time-of-flight state detection method provided by the embodiment of the present disclosure may obtain a plurality of groups of background coincidence events through different time windows based on the background inherent radiation phenomenon of LYSO. Based on different absorption characteristics of at least one β and γ particle, each pair of background coincidence events may be corrected. The TOF states of all crystals of the detector may be measured through the plurality of groups of background coincidence events, and the time of flight of the imaging device may be corrected through the energy-time mapping relationship, so as to obtain more accurate TOF information. By using the above TOF correction method, the correction time may be saved and the accuracy of TOF correction may be improved.

It should be noted that, the steps described in the above methods (for example, the method 700 for correcting the crystal position look-up table, the energy state detection method 1000, the method 1400 for determining the energy scale curve, the time-synchronization method 1600, and the time-of-flight state detection method 1900 of the imaging device) or shown in the flowchart of the drawings (for example, FIG. 7, FIG. 10, FIG. 14, FIG. 16, and FIG. 19) may be executed in a computer system such as a set of computer-executable instructions. And, although a logical sequence is shown in the flowchart, in some cases, the steps shown or described may be executed in a sequence different from that herein. Each module of the above devices (for example, the crystal position look-up table correction device 600, the energy state detection device 900, the energy scale curve determination device 1300, the time-synchronization device1500, and the time-of-flight state detection device 1800) may be either a functional module or a program module, which may be realized by a software or hardware. For modules implemented by a hardware, each of the above modules may be located in the same processor (e. g., the processing device 140); or the above modules may also be located in different processors in any combination.

The basic concepts have been described above. Obviously, for those skilled in the art, the above-detailed disclosure may be only an example and does not constitute a limitation of the present disclosure. Although it may be not explicitly stated here, those skilled in the art may make various modifications, improvements, and amendments to the present disclosure. Such modifications, improvements, and amendments are suggested in the present disclosure, so such modifications, improvements, and amendments still belong to the spirit and scope of the exemplary embodiments of the present disclosure.

Meanwhile, the present disclosure uses specific words to describe the embodiments of the present disclosure. For example, "one embodiment," and/or "some embodiments" mean a certain feature or structure related to at least one embodiment of the present disclosure. Therefore, it should be emphasized and noted that "one embodiment" or "an alternative embodiment" mentioned twice or more in different positions in the present disclosure does not necessarily refer to the same embodiment. In addition, certain features or structures in one or more embodiments of the present disclosure may be appropriately combined.

In addition, those skilled in the art may understand that various aspects of the present disclosure may be described through a number of patentable categories or situations, including any new and useful combination of processes, machines, products, or substances, or any new and useful improvements to them. Accordingly, various aspects of the present disclosure may be completely executed by hardware, software (including firmware, resident software, microcode, etc.), or a combination of hardware and software. The above hardware or software may be referred to as "data block," "module," "engine," "unit," "component" or "system". Further, various aspects of the present disclosure may be embodied in a computer product located in one or more computer-readable media, which includes computer-readable program coding.

The computer storage medium may comprise a propagation data signal containing a computer program encoding, for example, on a baseband or as part of a carrier wave. The transmitted signal may have multiple manifestations, including electromagnetic form, optical form, etc., or appropriate combination form. A computer storage medium may be any computer-readable medium other than a computer-readable storage medium, which may communicate, propagate, or transmit a program for use by connecting to an instruction execution system, device, or equipment. Program codes located on the computer storage medium may be transmitted through any suitable medium, including a radio, a cable, an optical fiber cable, an RF, or a similar media, or any combination of the above medium.

The computer program code required for the operation of each part of the present disclosure may be written in any one or more program languages, including object-oriented programming languages such as Java, Scala, Smalltalk, Eiffel, JADE, Emerald, C++, C #, and VB NET, Python, etc., conventional programming languages such as C language, Visual Basic, Fortran 2003, Perl, COBOL 2002, PHP, ABAP, dynamic programming languages such as Python, Ruby, Groovy, or other programming languages. The program code may be run completely on the user's computer, or as an independent software package on the user's computer, or partially on the user's computer, partly on the remote computer, or completely on the remote computer or server. In the latter case, the remote computer may connect with the user computer through any network form, such as a local area network (LAN) or a wide area network (WAN), or connect to an external computer (such as through the Internet), or in a cloud computing environment, or use as a service, such as software as a service (SaaS).

In addition, unless explicitly stated in the claims, the sequence of processing elements and sequences, the use of numbers and letters, or the use of other names described in the present disclosure are not configured to define the sequence of processes and methods in the present disclosure. Although the above disclosure has discussed some currently considered useful embodiments of the invention through various examples, it should be understood that such details are only for the purpose of explanation, and the additional claims are not limited to the disclosed embodiments. On the contrary, the claims are intended to cover all amendments and equivalent combinations that conform to the essence and scope of the embodiments of the present disclosure. For example, although the system components described above may be implemented by hardware devices, they may also be implemented only by software solutions, such as installing the described system on an existing server or mobile device.

Similarly, it should be noted that, in order to simplify the description disclosed in the present disclosure and thus help the understanding of one or more embodiments of the invention, the foregoing description of the embodiments of the present disclosure sometimes incorporates a variety of features into one embodiment, the drawings or the description thereof. However, this disclosure method does not mean that the object of the present disclosure requires more features than those mentioned in the claims. In fact, the features of the embodiments are less than all the features of the single embodiments disclosed above.

In some embodiments, numbers describing the number of components and attributes are used. It should be understood that such numbers used in the description of embodiments are modified by the modifier "about," "approximate" or "generally" in some examples. Unless otherwise stated, "approximately" or "generally" indicate that a ±20% change in the figure may be allowed. Accordingly, in some embodiments, the numerical parameters used in the description and claims are approximate values, and the approximate values may be changed according to the characteristics required by individual embodiments. In some embodiments, the numerical parameter should consider the specified significant digits and adopt the method of general digit reservation. Although the numerical fields and parameters configured to confirm the range breadth in some embodiments of the present disclosure are approximate values, in specific embodiments, the setting of such values may be as accurate as possible within the feasible range.

For each patent, patent application, patent application disclosure, and other materials cited in the present disclosure, such as articles, books, specifications, publications, documents, etc., the entire contents are hereby incorporated into the present disclosure for reference. Except for the present disclosure history documents that are inconsistent with or conflict with the contents of the present disclosure, and the documents that limit the widest range of claims in the present disclosure (currently or later appended to the present disclosure). It should be noted that in case of any inconsistency or conflict between the description, definitions, and/or use of terms in the supplementary materials of the present disclosure and the contents described in the present disclosure, the description, definitions, and/or use of terms in the present disclosure shall prevail.

Finally, it should be understood that the embodiments described in the present disclosure are only configured to illustrate the principles of the embodiments of the present disclosure. Other deformations may also fall within the scope of the present disclosure. Therefore, as an example rather than a limitation, the alternative configuration of the embodiments of the present disclosure may be regarded as being consistent with the teachings of the present disclosure. Accordingly, the embodiments of the present disclosure are not limited to those explicitly introduced and described in the present disclosure.

## Claims

1. A method for state detection of an imaging device, comprising:
obtaining a first background event of a crystal of a detector of an imaging device, the first background event being related to an inherent radiating particle of the crystal;
correcting a crystal position look-up table based on the first background event;
correcting an energy state of the imaging device;
obtaining a second background event of the crystal, the second background event being related to the inherent radiating particle of the crystal; and
correcting a state of time of flight of the detector based on the first background event and the second background event.

2. The method of claim 1, wherein
the obtaining a first background event of a crystal of a detector of the imaging device includes:
obtaining, based on a preset energy window, the first background event of the inherent radiating particle of the crystal received by the detector; and
the correcting a crystal position look-up table based on the first background event includes:
determining a single-event image based on the first background event; and
correcting the crystal position look-up table based on the single-event image.

3. The method of claim 1, wherein the correcting an energy state of the imaging device comprises:
correcting the energy state of the imaging device based on the first background event.

4. The method of claim 1, wherein the correcting an energy state of the imaging device comprises:
obtaining a third background event of the crystal, wherein the third background event is related to the inherent radiating particle of the crystal, and the third background event includes a background single event or a background coincidence event of the inherent radiating particle of the crystal received by the detector; and
correcting the energy state of the imaging device based on the third background event.

5. The method of claim 3 or 4, wherein the correcting an energy state of the imaging device comprises:
generating an energy spectrogram based on energy information of the first background event or the third background event;
determining at least one peak position in the energy spectrogram;
determining an energy correction state of the imaging device based on the at least one peak position in the energy spectrogram and a corrected peak position corresponding to the at least one peak position; and
correcting the energy state of the imaging device based on the energy correction state.

6. The method of claim 3 or 4, wherein the correcting the energy state of the imaging device comprises:
determining, based on the first background event or the third background event, at least two energy peak values associated with a nuclide decay of the crystal and ADC values corresponding to the at least two energy peak values; and
determining an energy scale curve of the imaging device based on the at least two energy peak values and the ADC values corresponding to the at least two energy peak values.

7. The method of claim 1, wherein the correcting a state of time of flight of the detector based on the first background event and the second background event includes:
determining a measured time of flight based on the first background event and the second background event; and
correcting a time of flight of the detector based on the state of time of flight of the imaging device reflected by the measured time of flight.

8. The method of claim 1, wherein the method further comprises:
generating an event time chart based on at least one of the first background event or the second background event;
determine a corresponding relationship between TDC values and time based on the event time chart; and
determining a TDC scale curve of the imaging device based on the corresponding relationship.

9. The method of claim 1, wherein the method further comprises:
obtaining a fourth background event of the crystal, the fourth background event being related to the inherent radiating particle of the crystal;
generating an event time chart based on the fourth background event;
determining a corresponding relationship between a TDC value and a time based on the event time chart; and
determining a TDC scale curve of the imaging device based on the corresponding relationship.

10. The method of claim 1, wherein the method further comprises:
determining, based on the first background event and the second background event, the measured time of flight and a theoretical time of flight; and
performing a time-synchronization on a detector module of the detector based on the measured time of flight and the theoretical time of flight.

11. A method for correcting a crystal position look-up table, wherein the method comprises:
obtaining a background event of a crystal of a detector of an imaging device, the background event being related to an inherent radiating particle of the crystal;
determining a single-event image based on the background event; and
correcting the crystal position look-up table of the imaging device based on the single-event image.

12. The method of claim 11, wherein the obtaining a background event of a crystal of a detector of an imaging device comprises:
determining an energy window of the detector of the imaging device, a value range of the energy window being within a clinical threshold value range of the energy window of the imaging device; and
obtaining, based on the energy window, the background event of the inherent radiating particle of the crystal received by the detector.

13. The method of claim 11, wherein the determining a single-event image based on the background event comprises:
determining a single-characteristic-energy-peak event based on the background event, wherein the single-characteristic-energy-peak event includes an event of at least one photon of 597 kev received by the detector; and
generating the single-event image based on the single-characteristic-energy-peak event.

14. The method of claim 11, wherein the correcting the crystal position look-up table of the imaging device based on the single-event image comprises:
obtaining, based on the single-event image, a corresponding pixel distribution of a position label of the crystal in the crystal position look-up table in the single-event image;
correcting the crystal position look-up table based on the corresponding pixel distribution of the position label of the crystal in the single-event image.

15. The method of claim 11, wherein the method further comprises:
determining whether a deviation related to the crystal position look-up table of the imaging device has occurred based on the background event.

16. A method of detecting an energy correction state, comprising:
obtaining a background event of a crystal of a detector of an imaging device, the background event being related to an inherent radiating particle of the crystal;
generating an energy spectrogram based on energy information of the background event;
determining at least one peak position in the energy spectrogram; and
determining the energy correction state based on the at least one peak position in the energy spectrogram and at least one corrected peak position corresponding to the at least one peak position in the energy spectrogram.

17. The method of claim 16, wherein the generating an energy spectrogram based on energy information of the background event comprises:
generating the energy spectrogram based on the energy information of the background event received by the detector in a single event mode, wherein the energy spectrogram includes at least one of a peak value of a full energy peak or a peak value of a single energy peak.

18. The method of claim 16, wherein the generating an energy spectrogram based on energy information of the background event comprises:
obtaining, based on at least one of a preset time window or a preset energy window, the background event of the inherent radiating particle of the crystal of the detector received by the detector in a coincident event mode, wherein a range of the preset time window is no less than a clinical threshold value range of a time window of the imaging device;
filtering particle energy information based on arrival time of at least one particle of the background event; and
generating the energy spectrogram based on the filtered particle energy information, wherein the energy spectrogram includes a peak value of a single energy peak.

19. The method of claim 16, wherein
the at least one peak position in the energy spectrogram includes at least one of a peak position of a full energy peak or a peak position of a single energy peak; and
the determining the energy correction state based on the at least one peak position in the energy spectrogram and at least one corrected peak position corresponding to the at least one peak position in the energy spectrogram includes:
determining a ratio of the at least one peak position in the energy spectrogram to the at least one corrected peak position; and
determining whether the energy correction state of the imaging device is abnormal based on the ratio.

20. The method of claim 16, wherein the corrected peak position corresponds to a peak position of at least one photon of 511 kev in the energy spectrum.

21. A method for determining an energy scale curve, comprising:
obtaining a background event of a crystal of a detector of an imaging device, the background event being related to an inherent radiating particle of the crystal;
determining, based on the background event, at least two energy peak values associated with a nuclide decay of the crystal and ADC values corresponding to the at least two energy peak values; and
determining the energy scale curve based on the at least two energy peak values and the ADC values corresponding to the at least two energy peak values.

22. The method of claim 21, wherein the determining at least two energy peak values associated with a nuclide decay of the crystal and ADC values corresponding to the at least two energy peak values based on the background event includes:
determining decay characteristic energy peak values of the nuclide of the crystal based on the background event;
obtaining an ADC value corresponding to the decay characteristic energy peak values by performing an analog-to-digital conversion on the decay characteristic energy peak values of the nuclide; and
selecting the at least two energy peak values in the decay characteristic energy peak values and the ADC values corresponding to the at least two energy peak values.

23. The method of claim 21, wherein:
the at least two energy peak values include an energy peak value of 307 kev and an energy peak value of 597 kev; and
the ADC values corresponding to the at least two energy peak values include an ADC value corresponding to the energy peak value of 307 kev and an ADC value corresponding to the energy peak value of 597 kev.

24. The method of claim 21, wherein the determining the energy scale curve based on the at least two energy peak values and the ADC values corresponding to the at least two energy peak values includes:
determining the energy scale curve by performing an interpolation and a fitting on the at least two energy peak values and the ADC values corresponding to the at least two energy peak values.

25. The method of claim 21, wherein the method further comprises:
determining an ADC value corresponding to an energy peak value of 511 kev based on the energy scale curve; and
correcting a particle energy received by the detector based on the ADC value corresponding to the energy peak value of 511 kev.

26. A time-synchronization method, comprising:
obtaining at least one background coincidence event of a crystal of a detector of an imaging device, the at least one background coincidence event being related to an inherent radiating particle of the crystal;
determining a measured time of flight and a theoretical time of flight based on information of the at least one background coincidence event; and
performing a time-synchronization on the detector based on the measured time of flight and the theoretical time of flight.

27. The method of claim 26, wherein
the information of the at least one background coincidence event includes: arrival time of two particles reaching the detector of each background coincidence event of the at least one background coincidence event and crystal positions corresponding to the two particles;
the determining a measured time of flight and a theoretical time of flight based on information of the at least one background coincidence event includes:
determining a measured time of flight of each background coincidence event based on the arrival time of the two particles of the background coincidence event; and
determining a theoretical time of flight of each background coincidence event based on the crystal positions corresponding to the two particles of the background coincidence event.

28. The method of claim 27, wherein the performing a time-synchronization on the detector based on the measured time of flight and the theoretical time of flight comprises:
determining a time difference based on the measured time of flight and the theoretical time of flight; and
performing the time-synchronization on the detector based on the time difference.

29. The method of claim 28, wherein the determining a time difference based on the measured time of flight and the theoretical time of flight comprises:
determining difference values between a plurality of measured values of time of flight and a plurality of theoretical values of time of flight based on the plurality of measured values of time of flight and the plurality of theoretical values of time of flight received by any pair of detector modules of the detector; and
determining a mean value of the difference values between the plurality of measured values of time of flight and the plurality of theoretical values of time of flight as the time difference.

30. The method of claim 28 or 29, wherein the performing the time-synchronization on the detector based on the time difference comprises:
adjusting a value of a counter of a detector module in any pair of detector modules of the detector in response to the time difference being greater than or equal to one clock cycle, and determining the adjusted value of the counter as a time reference standard; and
determining any one detector module in the pair of detector modules corresponding to the adjusted counter as a reference module, and performing the time-synchronization on other detector modules in the detector except the pair of detector modules based on the reference module.

31. A method for detecting a state of time of flight, comprising:
obtaining a background coincidence event of a crystal of a detector of an imaging device, the background coincidence event being related to an inherent radiating particle of the crystal;
determining a measured time of flight and a theoretical time of flight based on information of the background coincidence event; and
determining a state of time of flight of the imaging device based on the measured time of flight and the theoretical time of flight, wherein the state of time of flight reflects whether the crystal drifts.

32. The method of claim 31, wherein the obtaining a background coincidence event of a crystal of a detector of an imaging device comprises:
determining a time window and an energy window of the imaging device; and
obtaining, based on the time window and the energy window, the background event of the inherent radiating particle of the crystal received by the detector and the information of the background event,
wherein a range of the time window is no less than a clinical threshold value range of the time window of the imaging device, and a range of the energy window is no less than a clinical threshold value range of the energy window of the imaging device.

33. The method of claim 31, wherein
the information of the background coincidence event includes: arrival time of two particles reaching the detector of each background coincidence event of the background coincidence event and crystal positions corresponding to the two particles; and
the determining the measured time of flight and the theoretical time of flight based on information of the background coincidence event includes:
determining the measured time of flight of each background coincidence event based on the arrival time of the two particles of the background coincidence event; and
determining the theoretical time of flight of each background coincidence event based on the crystal positions corresponding to the two particles of the background coincidence event.

34. The method of claim 31, wherein the determining a state of time of flight of the imaging device based on the measured time of flight and the theoretical time of flight comprises:
in response to a difference between the measured time of flight and the theoretical time of flight exceeding a threshold value, determining that the crystal corresponding to the background coincidence event has drifted; and
correcting the time of flight of the detector based on the information of the background coincidence event to obtain a corrected time of flight.

35. The method of claim 34, wherein the correcting the time of flight of the detector based on the information of the background coincidence event to obtain a corrected time of flight comprises:
obtaining an energy-time mapping relationship, wherein the energy-time mapping relationship reflects a corresponding relationship between a particle energy and a time-of-flight offset;
determining, based on the energy-time mapping relationship, a time-of-flight offset corresponding to the energy of the two particles in each background coincidence event; and
correcting the time of flight of the detector based on the time-of-flight offset to obtain the corrected time of flight.

36. A state correction device for an imaging device, comprising:
an obtaining module, configured to obtain a first background event and a second background event of a crystal of a detector of an imaging device, the first background event and the second background event being related to an inherent radiation particle of the crystal particle; and
a correction module, configured to
correct a crystal position look-up table based on the first background event;
correct the energy state of the imaging device; and
correct the state of a time of flight of the detector based on the first background event and the second bottom event.

37. A computer device, comprising a memory, a processor, and a computer program stored on the memory and executable on the processor, wherein the processor implements the method described in any one of claims 1-35 when executing the computer program.

38. A computer-readable storage medium storing a computer program, wherein when the computer program is executed by a processor, the method described in any one of claims 1-35 is implemented.
